# EUROPEAN PATENT APPLICATION

(11) **EP 3 573 015 A1**
(43) Date of publication of application: **27.11.2019**
(21) Application number: 18742132.6
(22) Date of filing: 05.01.2018
(51) Int. Cl.: G06Q 50/22, A61G 12/00, G08B 25/04

(54) **ASSISTANCE METHOD AND ASSISTANCE SYSTEM**

(30) Priority: 19.01.2017 JP 2017007895
(71) Applicant: Konica Minolta, Inc., Tokyo 100-7015 (JP)
(72) Inventor: NISHIKADO, Masashi, Tokyo 100-7015 (JP); MIURA, Masanori, Tokyo 100-7015 (JP)
(74) Representative: Alton, Andrew
(86) International application number: PCT/JP2018/000087
(87) International publication number: WO 2018/135317

(57) **Abstract**

An assistance method used for an assistance system that assists a caregiver who gives care to a monitored person. The assistance system includes: a central processing unit that saves a care record; and a terminal device including an imaging unit that photographs an image, a display unit that displays the photographed image, and an input unit that inputs the care record, and communicably connected to the central processing unit. The assistance method includes: an imaging step of activating the imaging unit and photographing a care related image; a save selection screen display step of displaying, after photographing of the care related image, the photographed care related image and a save selection screen that prompts selection of whether to save the care related image in the central processing unit, on the display unit; an image transmission step of transmitting the care related image from the terminal device to the central processing unit after selection of saving of the care related image in the central processing unit; and a save-to-device step of saving the care related image as the care record in the central processing unit after transmission of the care related image.

## Description

### Technical Field

The present invention relates to an assistance method used for an assistance system for assisting a caregiver who gives care to a monitored person, and an assistance system.

### Background Art

In an aging society such as Japan in recent years, the number of people (persons requiring care) in need of nursing or care due to illness, injury or old age is estimated to increase further in the future. Such a person requiring care is admitted by a hospital or facility such as a care home and is taken care of by caregivers. At facilities such as hospitals and care homes, the caregivers who cares for the person requiring care confirms safety of the person requiring care by regular patrol. In terms of safety confirmation, a single person, that is, a person living alone, can also be a person requiring care.

However, compared with the daytime work hours, the number of caregivers decreases and the work load per person increases during the evening work hours and night work hours, leading to the need to reduce the work load. For this reason, in recent years, there have been researched and developed assistance systems to assist caregivers who care for a monitored person as a person requiring care (refer to Patent Literature 1). For example, a system described in Patent Literature 1 above has a configuration in which a caregiver who cares for a person requiring care creates a record card (care record) that records content of care, and photographs and saves the created care record with a terminal device. Furthermore, the above Patent Literature 1 describes operation of photographing an image of an affected part (care related image being an image relevant to the care) of the person requiring care and saving the photographed care related image (Paragraphs [0012] and [0016]) in Patent Literature 1).

### Citation List

### Patent Literature

Patent Literature 1: JP 2014-215687 A

### Summary of Invention

The above-described Patent Literature 1 includes no description of specific saving procedure of the care related image. However, it would be desirable, in the system in Patent Literature 1 described above, to enable saving care related images as care records in a simple procedure.

The present invention has been made in view of the above-described circumstances, and an object thereof is to provide an assistance method and an assistance system capable of saving a care related image as a care record in a simple procedure.

In order to achieve the above-described object, an assistance control method according to one aspect of the present invention is an assistance method used for an assistance system that assists a caregiver who cares for a monitored person,
the assistance system including:
a central processing unit that saves a care record that records content of care given to the monitored person by the caregiver; and
a terminal device including an imaging unit that photographs an image, a display unit that displays the image photographed by the imaging unit, and an input unit that inputs the care record, and communicably connected to the central processing unit,
the method including:
   an imaging step of activating the imaging unit and photographing a care related image being an image related to the content of the care given by the caregiver;
   a save selection screen display step of displaying, after photographing of the care related image, the photographed care related image and a save selection screen that prompts selection of whether to save the care related image in the central processing unit, on the display unit;
   an image transmission step of transmitting the care related image from the terminal device to the central processing unit after selection of saving of the care related image in the central processing unit in accordance with the save selection screen; and
   a save-to-device step of saving the care related image as the care record in the central processing unit after transmission of the care related image.

Advantages and features obtained by one or more embodiments of the invention will be sufficiently understood from the detailed description given below and the accompanying drawings. These detailed descriptions and the accompanying drawings are given by way of example only and are not intended to be a definition limited to the present invention.

### Brief Description of Drawings

Fig. 1 is a view illustrating a configuration of an assistance system in the present embodiment.
Fig. 2 is a block diagram illustrating a configuration of a management server device.
Fig. 3 is a diagram illustrating a configuration of a server side monitoring information table.
Fig. 4 is a diagram illustrating the configuration of an inter-device information table.
Fig. 5 is a diagram illustrating a configuration of a server side sensor information table.
Fig. 6 is a view illustrating an example of a meal care recording format screen.
Fig. 7 is a view illustrating an example of a morning care recording format screen.
Fig. 8 is a view illustrating an example of a bowl movement care recording format screen.
Fig. 9 is a diagram illustrating a configuration of a format information table.
Fig. 10 is a diagram illustrating a configuration of a server side care record information table.
Fig. 11 is a block diagram illustrating a configuration of a mobile terminal device.
Fig. 12 is a view illustrating an example of a login screen.
Fig. 13 is a view illustrating an example of a user list selection screen.
Fig. 14 is a view illustrating an example of a passcode input screen.
Fig. 15 is a view illustrating an example of a standby screen.
Fig. 16 is a sequence diagram schematically illustrating a first example of care related image recording operation.
Fig. 17 is a view illustrating an example of a sub-menu screen.
Fig. 18 is a view illustrating an example of a photographing screen.
Fig. 19 is a view illustrating an example of a save selection screen.
Fig. 20 is a view illustrating an example of a target person selection screen.
Fig. 21 is a view illustrating an example of a format selection screen.
Fig. 22 is a view illustrating an example in which a thumbnail image is displayed on a format selection screen.
Fig. 23 is a view illustrating an example of a save selection screen.
Fig. 24 is a sequence diagram schematically illustrating a second example of the care related image recording operation.
Fig. 25 is a sequence diagram schematically illustrating a second example of the care related image recording operation.
Fig. 26 illustrates an example of the save selection screen.
Fig. 27 is a sequence diagram schematically illustrating a third example of the care related image recording operation.
Fig. 28 is a sequence diagram schematically illustrating the third example of the care related image recording operation.
Fig. 29 is a diagram illustrating an example of the save selection screen.
Fig. 30 is a sequence diagram schematically illustrating a fourth example of the care related image recording operation.
Fig. 31 is a sequence diagram schematically illustrating the fourth example of the care related image recording operation.
Fig. 32 is a view illustrating an example of a monitoring information screen.
Fig. 33 is a view illustrating an example of a nurse call reception screen.
Fig. 34 is a block diagram illustrating a configuration of a fixed terminal device.
Fig. 35 is a view illustrating an example of a viewer screen of a care record.

### Description of Embodiments

### (Findings underlying the present invention)

First, findings underlying the present invention will be described. A care record that records the content of care given by a caregiver for the person requiring care is basically created by text input. For example, injuries or the like caused by an accident such as a tumbling are represented as a picture of texts or a hand writing image and recorded. In addition, care records related to the amount of meal, the amount of bowel movements, and the condition of the caregiver are also created by text input. This might result in low accuracy in care records and taking time for creating the care records.

In contrast, in the system described in Patent Literature 1, it is possible, as described above, to photograph an image of an affected part of the person requiring care (a care related image being an image related to care) and save the photographed care related image. The above Patent Literature 1, however, includes no specific procedure of saving care related images. To overcome this issue, the present inventors have conceived of an invention that enables easy saving of care related images photographed at various timings, as care records.

### (Embodiments)

One or more embodiments of the present invention will now be described with reference to the drawings. The scope of the invention is not limited to the embodiments disclosed. In the drawings, the same reference numerals denote the same components, and description thereof will be omitted as appropriate. This description uses a reference numeral without suffix for the use as a generic term, and uses a reference numeral with suffix for indicating individual configurations.

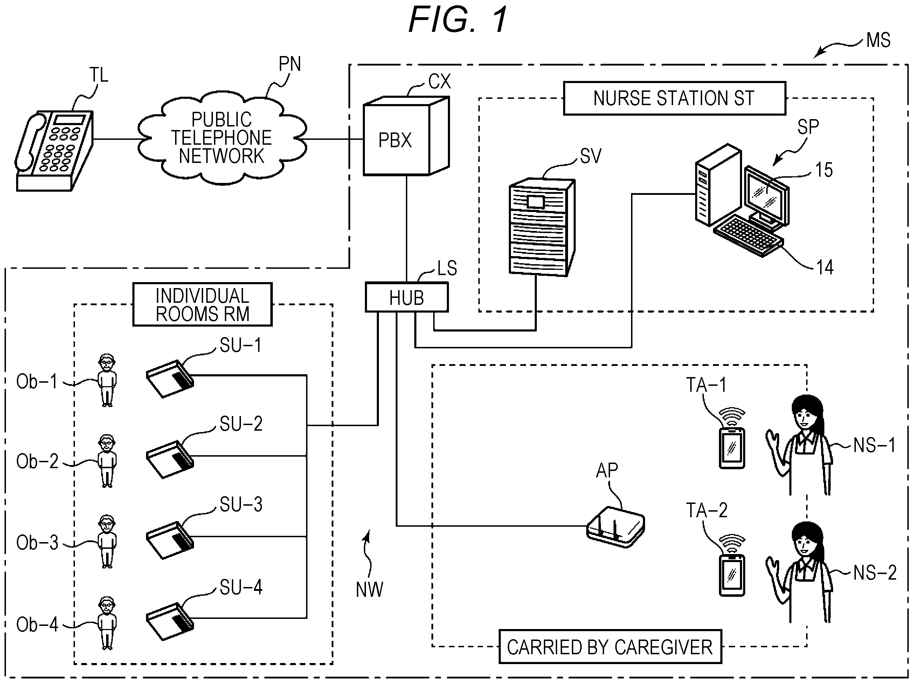

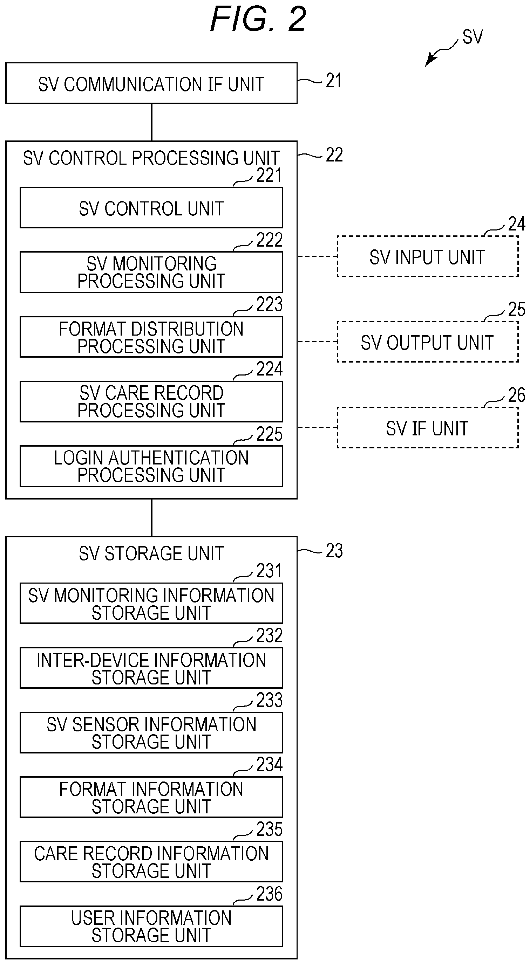

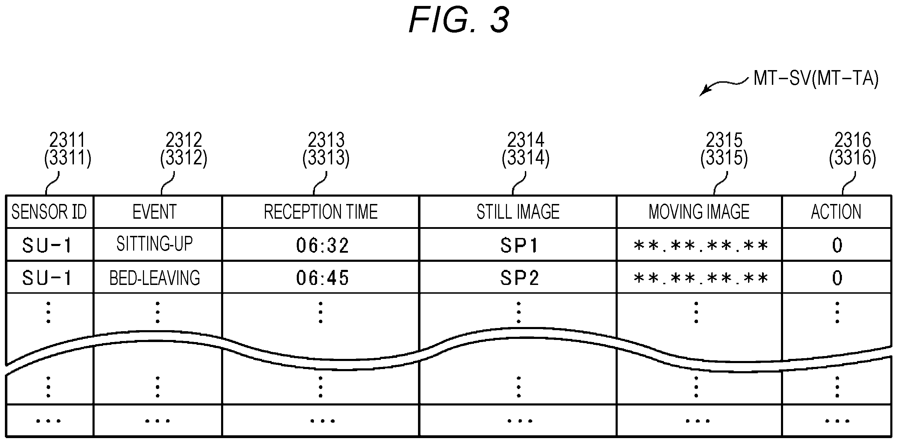

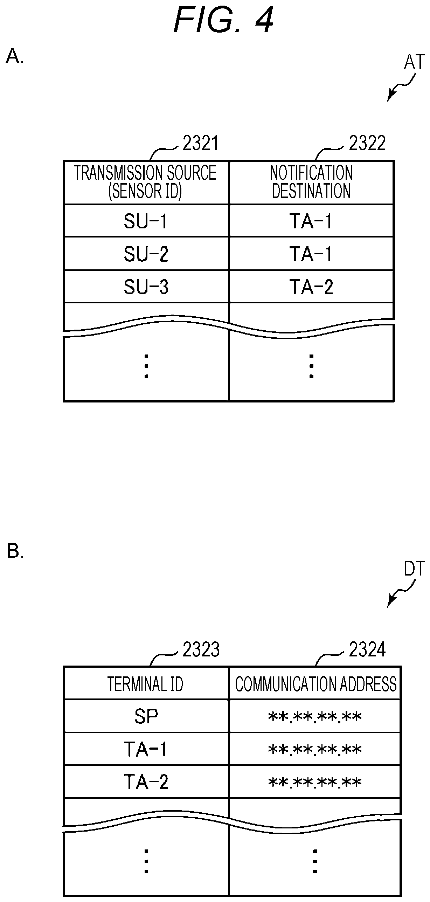

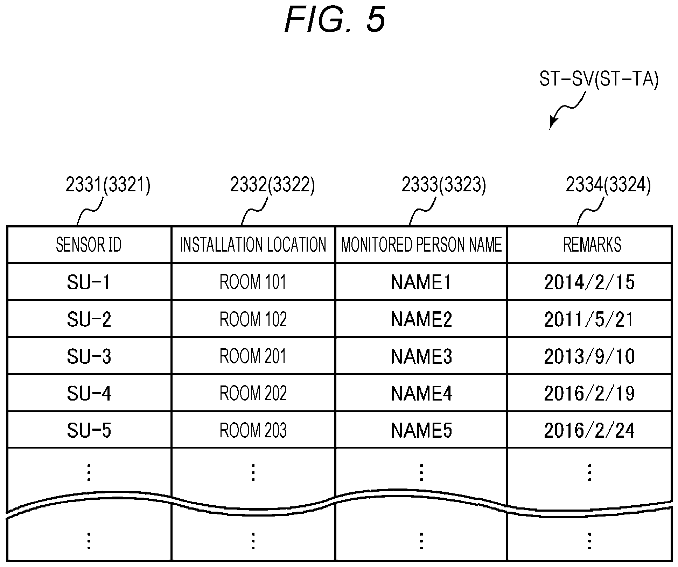

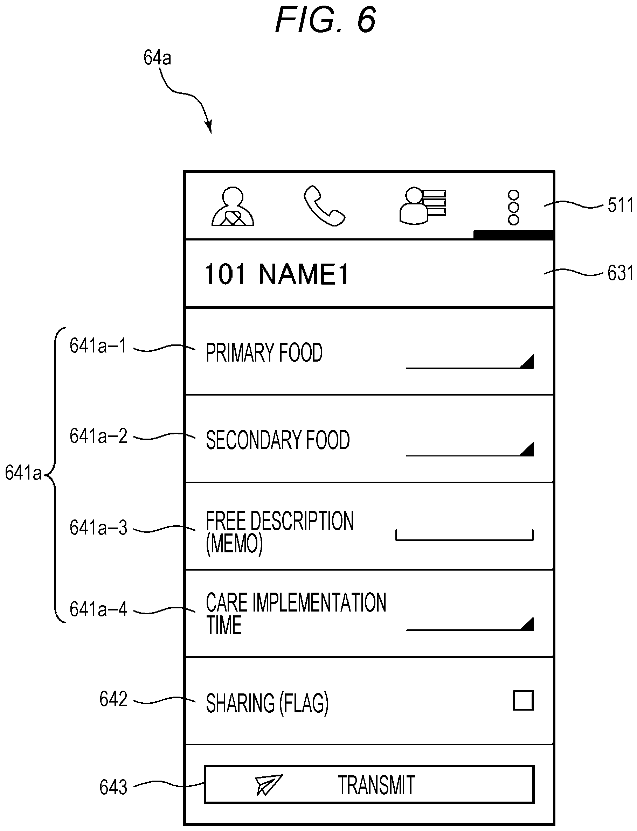

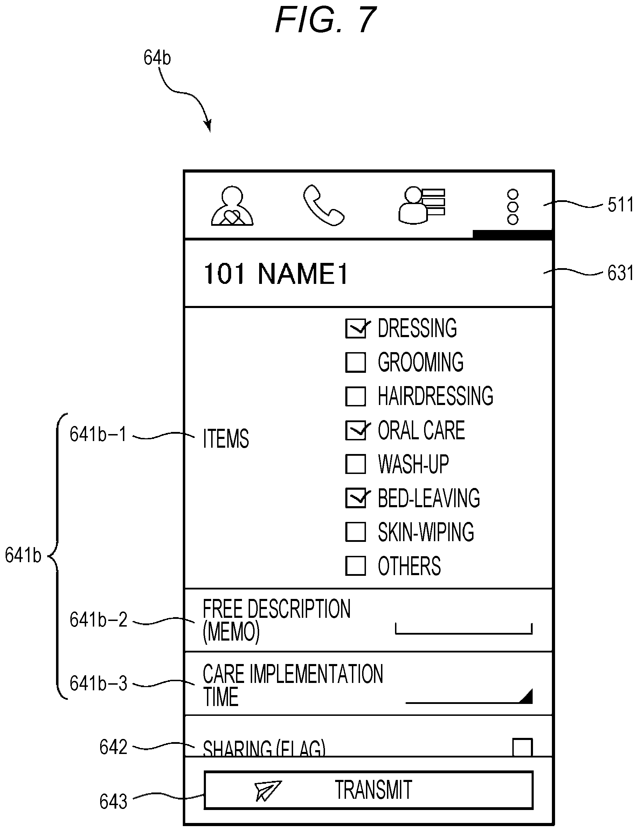

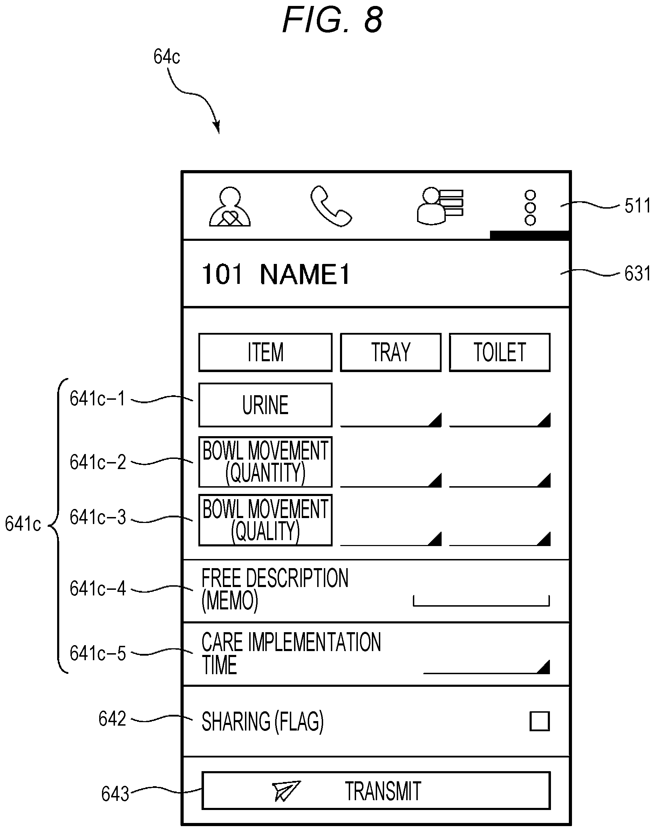

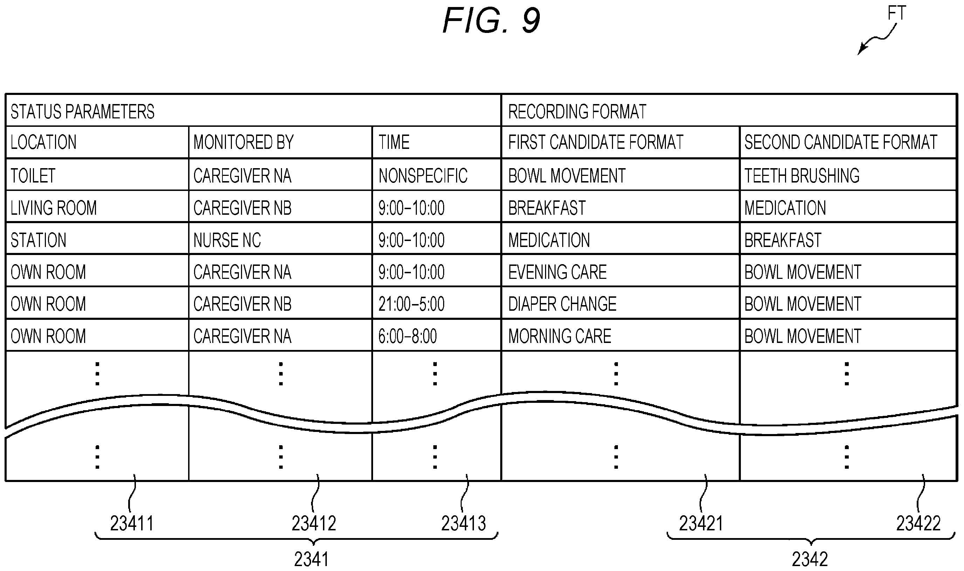

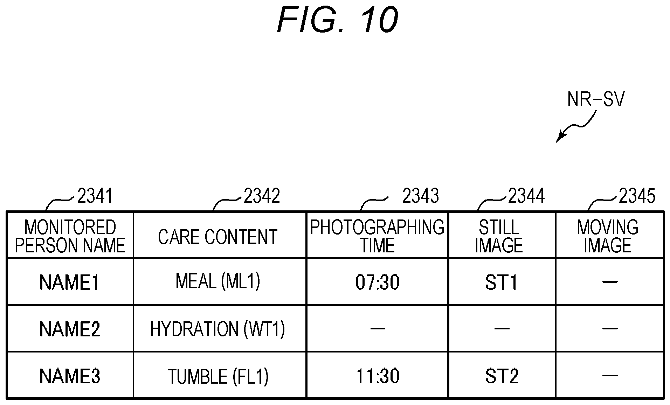

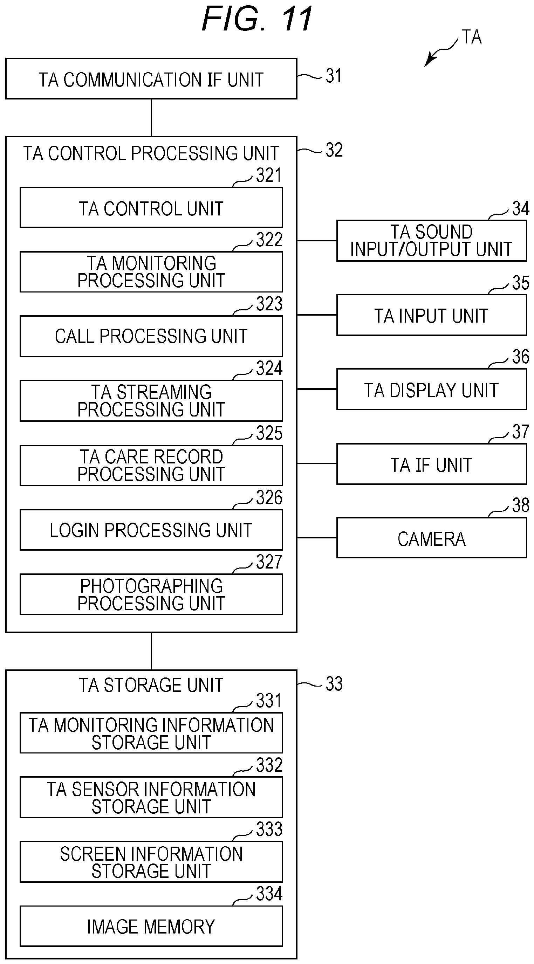

Fig. 1 is a view illustrating a configuration of an assistance system in the present embodiment. Fig. 2 is a block diagram illustrating a configuration of a management server device included in the assistance system. Fig. 3 is a diagram illustrating the configuration of a server side monitoring information table stored in the management server device. Fig. 4 is a diagram illustrating configurations of inter-device information tables stored in the management server device. Fig. 4A illustrates a configuration of a notification destination correspondence relationship information table out of the inter-device information table. Fig. 4B illustrates a configuration of a communication address correspondence relationship information table out of the inter-device information table. Fig. 5 is a diagram illustrating a configuration of a server side sensor information table stored in the management server device. Fig. 6 is a diagram illustrating an example of a meal care recording format screen displayed on a mobile terminal device. Fig. 7 is a view illustrating an example of a morning care recording format screen displayed on a mobile terminal device. Fig. 8 is a view illustrating an example of a bowl movement care recording format screen displayed on a mobile terminal device. Fig. 9 is a diagram illustrating a configuration of a format information table stored in the management server device. Fig. 10 is a diagram illustrating a configuration of a server side care record information table stored in the management server device. Fig. 11 is a block diagram illustrating a configuration of a mobile terminal device included in the assistance system.

An assistance system MS of the present embodiment illustrated in Fig. 1 assists a caregiver who cares (nurses or give care to) a monitored person Ob (person requiring care) as a target of monitoring. The monitored person Ob can also be referred to as a watch-over target person who is a target of watching over.

More specifically, for example, as illustrated in Fig. 1, the assistance system MS in the present embodiment includes one or more sensor devices SU (SU-1 to SU-4), a management server device SV, and a fixed terminal device SP, a plurality of mobile terminal devices TA (TA-1 and TA-2), and a private branch exchange (PBX) CX. These components are communicably connected with wired or wireless communication via a network NW such as a local area network (LAN). The network NW may have relay devices such as repeaters, bridges and routers for relaying communication signals.

In the example illustrated in Fig. 1, the plurality of sensor devices SU-1 to SU-4, the management server device SV, the fixed terminal device SP, the plurality of mobile terminal devices TA-1 and TA-2, and the private branch exchange CX are communicably connected with each other by a wired/wireless mixed network (for example, a LAN according to the IEEE 802.11 standard) NW including a concentrator LS of an L2 switch and an access point AP. More specifically, the plurality of sensor devices SU-1 to SU-4, the management server device SV, the fixed terminal device SP, and the private branch exchange CX are connected to the concentrator LS, while the plurality of mobile terminal devices TA-1 and TA-2 are connected to the concentrator LS via the access point AP.

Although the number of concentrators LS and the number of access points AP are each illustrated as one in the example illustrated in Fig. 1, each of them may be provided in plural. The network NW constructs a so-called intranet by using an Internet protocol group such as a transmission control protocol (TCP) and an Internet protocol (IP).

The assistance system MS is installed at an appropriate place in accordance with the monitored person Ob. Examples of the monitored person Ob include a person who needs nursing due to illness or injury, a person who needs care due to a decline in physical ability, and a single person who is living alone. In particular, from the viewpoint of enabling early detection and early handling, the monitored person Ob would preferably be a person who needs a discovery in a case where a predetermined unfavorable event such as an abnormal condition occurs in the person. Accordingly, the assistance system MS is preferably installed in buildings such as hospitals, care homes, and dwellings according to the type of monitored person Ob. In the example illustrated in Fig. 1, the assistance system MS is installed in a building of a care house including a plurality of rooms RM in which a plurality of monitored persons Ob reside and a plurality of rooms such as a nurse station ST.

The sensor device SU is a device that includes a communication function to communicate with other devices SV, SP, and TA via the network NW, detects a predetermined event related to the monitored person Ob, and notifies the management server device SV of the detected event. The predetermined event is preferably a predetermined event that needs to be handled, and is a predetermined behavior and a nurse call (NC) preliminarily set on the monitored person Ob in the present embodiment, for example.

More specifically, the sensor device SU includes: a communication interface circuit (for example, a LAN card or the like) for communicating with other device SV, SP, and TA via the network NW; an image sensor that photographs an image of the monitored person Ob or the like and generates a photographed image; a behavior detection processing circuit that detects a predetermined behavior preliminarily set in the monitored person Ob as an example of the predetermined event on the basis of the image output from the image sensor and notifies the detected behavior to the management server device SV; a nurse call processing circuit that receives a nurse call as another example of the predetermined event and notifies the management server device SV of the nurse call; a call processing circuit that performs voice calling with the terminal devices SP, TA, or the like; an image transmission processing circuit that transmits images including still images and moving images output from the image sensor to predetermined other devices SV, SP, and TA; a control circuit for controlling these circuits; and its peripheral circuits.

In the present embodiment, examples of the predetermined behavior include four behaviors of: sitting-up, that is, sitting-up of the monitored person Ob; bed-leaving, that is, leaving of the monitored person Ob from bedding; falling, that is, falling of the monitored person Ob off the bed; and tumbling, that is, tumbling of the monitored person Ob. The behavior detection processing circuit detects the head of the monitored person Ob on the basis of a target image being an image captured by the image sensor, for example, and detects sitting-up, bed-leaving, falling and tumbling of the monitored person Ob on the basis of a temporal change in the size of the detected head of the monitored person Ob.

More specifically, first, preliminarily stored are: the location area of the bedding; a first threshold Th1 for discriminating between the size of the head in the lying position and the size of the head in the sitting position in the location area of bedding; a second threshold Th2 for discriminating whether the size is the size of the head in the standing position within a room RM excluding the location area of the bedding; and a third threshold Th3 for discriminating whether the size is the size of the head in the lying position in the room RM excluding the location area of the bedding.

Subsequently, the behavior detection processing circuit extracts a moving body area from the target image as a person area of the monitored person Ob using the background subtraction method or the frame subtraction method, for example, and extracts a head area of the monitored person Ob from the extracted moving body area using, for example, circular or oval Hough transformation, or pattern matching using a prepared head model, or a neural network learned for head detection, and then, detects sitting-up, bed-leaving, tumbling, or falling from the extracted head position and size.

For example, in a case where the position of the extracted head is within the location area of the bedding, and the size of the extracted head has a temporal change from the size of the lying position to the size of the sitting position by using the first threshold Th1, the behavior detection processing circuit determines that the monitored person Ob is in the sitting-up and detects this as sitting-up.

For example, in a case where the position of the extracted head has a temporal change from within the location area of the bedding to the outside the location area of the bedding and the size of the extracted head has a temporal change from a certain size to the size of the standing position by using the second threshold Th2, the behavior detection processing circuit determines that the monitored person Ob is leaving the bed and detects this as bed-leaving.

For example, in a case where the position of the extracted head has a temporal change from within the location area of the bedding to the outside the location area of the bedding and the size of the extracted head has a temporal change from a certain size to the size of the lying position by using the third threshold Th3, the behavior detection processing circuit determines that the monitored person Ob has fallen off the bed and detects this as falling.

For example, in a case where the position of the extracted head is within the room RM excluding the location area of the bedding, and the size of the extracted head has a temporal change from a certain size to the size of the lying position by using the third threshold Th3, the behavior detection processing circuit determines that the monitored person Ob has tumbled and detects this as the tumbling.

The predetermined event is notified from the sensor device SU to the management server device SV by a first event notification communication signal. The first event notification communication signal contains the sensor ID of its own device and event information representing the content of the event. The sensor ID (sensor identifier) is an identifier for determining and identifying the sensor device SU. In the present embodiment, the event information is one or more of sitting-up, bed-leaving, falling, tumbling, or a nurse call.

The first event notification communication signal may contain an image captured by the image sensor. In particular, in a case where the event information is any of sitting-up, bed-leaving, falling, or tumbling, it would be preferable to contain an image used to detect the predetermined behavior (last image in a case where the detection is performed by a plurality of images, for example) in the event information. The image may be at least one of a still image or a moving image. In the present embodiment, a still image is first notified, and a moving image is then distributed in response to a user's request, as described below. Alternatively, a moving image may be distributed first, or a still image and a moving image may be transmitted, and then, the still image and the moving image may be displayed on the terminal devices SP and TA by screen split.

Fig. 1 illustrates four sensors, namely first to fourth sensor devices SU-1 to SU-4 as an example. The first sensor device SU-1 is installed in a room of a monitored person Ob-1. The second sensor device SU-2 is installed in a room of a monitored person Ob-2. The third sensor device SU-3 is installed in a room of a monitored person Ob-3. The fourth sensor device SU-4 is installed in a room of a monitored person Ob-4.

The management server device SV has a communication function of communicating with other devices SU, TA, and SP via the network NW. After receiving a notification of the predetermined event from the sensor device SU, the management server device SV manages monitoring information being information related to monitoring of the monitored person Ob, and then, notifies the predetermined event to predetermined terminal devices SP and TA. The management server device SV supplies data to the client in response to the request from a client (terminal device SP, TA, etc. in the present embodiment) and performs overall management of the assistance system MS. In the present embodiment, examples of the monitoring information include the predetermined event (the type of a predetermined behavior detected by the sensor device SU and the nurse call received by the sensor device SU in the present embodiment), images including a still image or a moving image of the monitored person Ob, the time of reception of the notification, or the like.

Subsequently, in the present embodiment, the management server device SV stores a plurality of recording formats according to the type of care given by the monitoring person (user) for the monitored person Ob. When a request for the recording format is received from the terminal devices SP or TA, the management server device SV returns the recording format based on a status parameter in the terminal device SP or TA as a transmission source, to the terminal device SP or TA as the transmission source. After reception of a care record using the recording format, the management server device SV stores the care record.

After reception of a care related image, that is, an image related to care photographed by the mobile terminal device TA and transmitted from the mobile terminal device TA, the management server device SV (corresponding to an example of the central processing unit) stores the received care related image as a care record in association with the monitored person Ob and the type of care corresponding to the care related image. For example, as illustrated in Fig. 2, such a management server device SV includes a server side communication interface unit (SV communication IF unit) 21, a server side control processing unit (SV control processing unit) 22, and a server side storage unit (SV storage unit) 23.

The SV communication IF unit 21 is a communication circuit connected to the SV control processing unit 22 and configured to perform communication under the control of the SV control processing unit 22. The SV communication IF unit 21 generates a communication signal containing data to be transferred input from the SV control processing unit 22 in accordance with a communication protocol used in the network NW of the assistance system MS, and then, transmits the generated communication signal to the other devices SU, SP and TA via the network NW.

The SV communication IF unit 21 receives a communication signal from other devices SU, SP, and TA via the network NW, takes out data from the received communication signal, and converts the extracted data into data having a format that can be processed by the SV control processing unit 22, and then outputs the converted data to the SV control processing unit 22. The SV communication IF unit 21 includes a communication interface circuit conforming to the IEEE 802.11 standard, for example.

The SV storage unit 23 is a circuit connected to the SV control processing unit 22 and configured to store various predetermined programs and various predetermined data under the control of the SV control processing unit 22. Example of various predetermined programs include an SV control program for controlling individual portions of the management server device SV according to the function of the individual portions; an SV monitoring processing program for executing predetermined information processing related to monitoring of the monitored person Ob; a format distribution processing program that receives a request for a recording format from the terminal device SP or TA and then returns a recording format based on status parameters in the terminal device SP or TA as the transmission source to the terminal device SP or TA as the transmission source; an SV care record processing program that receives a care record using a recording format and then stores the care record; and a control processing program such as a login authentication processing program that authenticates the login of the mobile terminal device TA.

Examples of the various predetermined data include data necessary for execution of individual program, such as: a server identifier (server ID) of the own device for determining the management server device SV and identifying the management server device SV; the monitoring information of the monitored person Ob; inter-device information representing information between devices SU, SP, and TA, such as notification destinations of the predetermined events; sensor information regarding the sensor device SU; format information regarding the recording format; and care record information being information regarding a care record.

The SV storage unit 23 includes a read only memory (ROM) which is a nonvolatile memory element, and an electrically erasable programmable read only memory (EEPROM) which is a rewritable nonvolatile storage element, for example. The SV storage unit 23 includes a random access memory (RAM) serving as a working memory of the SV control processing unit 22 for storing data or the like generated during execution of the predetermined program.

Additionally, in order to individually store the monitoring information, the inter-device information, the sensor information, the format information and the care record information individually, the SV storage unit 23 functionally includes: a server side monitoring information storage unit (SV monitoring information storage unit) 231; an inter-device information storage unit 232; a server side sensor information storage unit (SV sensor information storage unit) 233; a format information storage unit 234; a care record information storage unit 235 and a user information storage unit 236.

The SV monitoring information storage unit 231 stores monitoring information of the monitored person Ob, transmitted and received between each of the devices SU, SP, and TA. More specifically, in the present embodiment, the SV monitoring information storage unit 231 stores, as the monitoring information, the sensor ID, event information (sitting-up, bed-leaving, falling, tumbling and a nurse call, in the present embodiment), reception time, target images including still images and moving images being images captured by an image sensor, and the presence or absence of handling in association with one another, on the basis of individual information contained in the communication signal such as the first event notification communication signal.

This monitoring information is stored in the SV monitoring information storage unit 231 in the form of a table in the present embodiment. The server side monitoring information table (SV monitoring information table) MT-SV that registers this monitoring information includes, for example, as illustrated in Fig. 3, a sensor ID field 2311 for registering the sensor ID contained in the communication signal received from each of devices SU, SP, and TA; an event field 2312 for registering event information contained in the received communication signal; a reception time field 2313 for registering the reception time of the received communication signal; a still image field 2314 for registering the still image contained in the received communication signal; a moving image field 2315 for registering a communication address (for example, an IP address or the like) of the sensor device SU corresponding to the sensor ID contained in the received communication signal, as a live moving image acquisition destination; and an action field 2316 for registering the presence or absence of the reception of the action taken for the event information contained in the received communication signal. Each of the received communication signals, that is, each of the events includes a record. For example, the still image field 2314 may register image data of a still image, for example, and may register a file name of image data of a still image.

As described below, the action field 2316 registers an action flag being a flag that indicates whether the terminal devices SP and TA have received an intention ("Action") to take action for the event information contained in the received communication signal. For example, in the present embodiment, the action field 2316 registers either an action flag "1" or "0". The action flag "1" indicates that the terminal device SP or TA has received an intention ("Action") to take action for the event information (event information registered in the event field 2312) contained in the received communication signal. The action flag "0" indicates that the terminal device SP or TA has not received the intention ("Action") to take action for the event information contained in the received communication signal. By default, an action flag "0" indicating non-reception is registered to the action field 2316.

Note that in a case where the first event notification communication signal contains the detection time at which the predetermined behavior has been detected or the nurse call reception time at which the nurse call has been received, the reception time field 2313 may register the detection time or the nurse call reception time in place of the reception time.

In the present embodiment, the inter-device information storage unit 232 stores, as the inter-device information, a notification destination correspondence relationship that is a correspondence relationship between the sensor ID that is a transmission source, the sensor ID indicating a notification destination such as a first event notification communication signal transmitted from the sensor device SU with the terminal ID being a notification destination, and a communication address correspondence relationship that is a correspondence relationship between the sensor ID of the device SU, and the terminal ID of the device SP or TA and their communication addresses, or the like. The terminal ID is a terminal identifier for determining the terminal device SP or TA and identifying the terminal device SP or TA.

The notification destination correspondence relationship and the communication address correspondence relationship are individually stored in the inter-device information storage unit 232 in the form of a table in the present embodiment. As illustrated in Fig. 5A, a notification destination correspondence relationship information table AT that registers the notification destination correspondence relationship includes: a transmission source field 2321 for registering the sensor IDs of the sensor device SU as the transmission source; and a notification destination field 2322 for registering the terminal ID of the terminal devices SP and TA as a transmission destination to which a communication signal notified from the sensor device SU corresponding to the sensor ID registered in the transmission source field 2321 is transmitted. Each of the sensor IDs (that is, each of the sensor devices SU) has a record.

As illustrated in Fig. 5B, a communication address correspondence relationship information table DT for registering the communication address correspondence relation includes, for example: a terminal ID field 2323 for registering terminal IDs of the terminal devices SP and TA; and a communication address field 2324 for registering communication addresses of terminal devices SP and TA corresponding to the terminal ID registered in the terminal ID field 2323. Each of the terminal IDs (that is, each of the terminal devices SP and TA) has a record.

Each of the sensor ID, the server ID and the terminal ID may be, for example, a serial number or the like including a predetermined symbol string, or may be, for example, a communication address. In a case where each of the sensor ID, server ID and terminal ID is a communication address, the communication address correspondence relationship can be omitted. The notification destination correspondence relationship has a configuration in which a plurality of sensor devices SU is associated with one terminal device SP or TA.

The SV sensor information storage unit 233 stores the sensor information. In the present embodiment, the sensor information is information regarding the sensor device SU, and is information that associates the sensor ID of the sensor device SU with a monitored person name, that is, the name of the monitored person Ob.

In the present embodiment, such sensor information is stored in the format of the table in the SV sensor information storage unit 233. More specifically, as illustrated in Fig. 5, for example, the server side sensor information table (SV sensor information table) ST-SV that registers sensor information includes: a sensor ID field 2331 for registering a sensor ID; an installation location field 2332 for registering an installation location of the sensor device SU having the sensor ID registered in the sensor ID field 2331; a monitored person name field 2333 for registering the monitored person name of the monitored person Ob monitored by the sensor device SU having the sensor ID registered in the sensor ID field 2331 (that is, the monitored person Ob present at an installation location of the sensor device SU having the sensor ID registered in the sensor ID field 2331); and a remarks field 2334 that registers remarks regarding the sensor device SU having the sensor ID registered in the sensor ID field 2331 and its installation location and its monitored person Ob. Each of the sensor IDs (that is, each of the sensor devices SU) has a record.

The format information storage unit 234 stores the format information. The format information is information regarding a recording format for inputting the content of implemented care as a care record. The recording format is prepared in plurality as a predetermined mode (format, input form) defined in advance, according to the type of care.

The types of care may vary depending on care practices such as nursing practice and care-giving practice. For example, the types of care include regular care and location-dependent care. More specifically, examples of regular care include: morning care (changing clothes, washing and wearing denture, or the like), meal care (breakfast care, lunch care, dinner care), snacking care, medication care, tooth brushing care, hydration care, vital check, evening care (changing clothes, tooth brushing care, denture removal, bed transfer, or the like), patrols, and position change, implemented on a daily basis; and bath care, rehabilitation, recreation, examination by room visit, body cleansing (bed bath), weight measurement, or the like performed a certain day of the week or of the month. The care that depends on location is bowl movement care, or the like.

These cares depend on locations such as a living room, a room RM, toilet, a bath room, and nurse stations, or depends on monitoring persons such as nurses and caregivers etc. or a care person in charge. The recording format is prepared in advance according to the content of care, for example, and includes a recording format for morning care, a recording format for meal care, a recording format for snack care, a recording format for medication care, a recording format for tooth care, and a recording format for hydration care.

A meal care recording format screen 64a is a screen for displaying a recording format for meal care, and is a screen for inputting the content of the implemented meal care as a care record. For example, as illustrated in Fig. 6, the meal care recording format screen 64a includes: a menu bar area 511; a target person name display area 631 for displaying a target person name; a care record input area 641a that displays input items to be input as content of care for inputting the content of care according to the input item as a care record; a sharing check box (□) 642 for inputting the setting as to whether this care record can be referred to from the fixed terminal device SP and other mobile terminal device TA; and a "transmit" button 643.

In the recording format for meal care, for example, in the present embodiment, the input items are the intake amount of primary food, the intake amount of the secondary food, the memo and the care implementation time. In order to input these items, the care record input area 641a includes: a primary food amount input area 641a-1 for inputting the amount of primary food; a secondary food amount input area 641a-2 for inputting the amount of a secondary food; a free description input area 641a-3 for inputting a memo; and a care implementation time input area 641a-4 for inputting the time when the monitored person Ob received care.

In the sharing check box 642, when the check mark input operation is received, "check mark" is displayed in the sharing check box 642, and there is an input of instruction to make the setting to enable reference of the content input on a recording format screen 64 (in the example illustrated in Fig. 6, the meal care recording format screen 64a) from the fixed terminal device SP and other mobile terminal device TA. In contrast, when the check mark input operation has not been received, nothing is displayed in the sharing check box 642, and there is an input of instruction to make the setting to disable reference of the content input on the recording format screen 64 (in the example illustrated in Fig. 6, the meal care recording format screen 64a) from the fixed terminal device SP and other mobile terminal device TA.

The "transmit" button 643 is a button to input an instruction to transmit the content (content of meal care in the example illustrated in Fig. 6) input to the recording format screen 64 (or the meal care recording format screen 64a in the example illustrated in Fig. 6) to the management server device SV.

A morning care recording format screen 64b illustrated in Fig. 7 is a screen for displaying a recording format for morning care and is a screen for inputting the content of the implemented morning care as a care record. For example, as illustrated in Fig. 7, the morning care recording format screen 64b includes: a menu bar area 511; a target person name display area 631; and a care record input area 641b that displays input items as to be input as content of care for inputting the content of care according to the input item as a care record; a sharing check box (□) 642; and a "transmit" button 643.

For example, in the present embodiment, the recording format for morning care includes, as the input items, dressing, grooming, hairdressing, oral care, wash-up, bed-leaving, skin-wiping, other items, memos, and care implementation time. In order to input these, the care record input area 641b includes an item area 641b-1, a free description input area 641b-2 for memo purpose, and a care implementation time input area 641b-3 for inputting time when the care is given to the monitored person Ob. The item area 641b-1 further includes: a dressing input check box (□) for inputting presence/absence of dressing; a grooming input check box (□) for inputting presence/absence of grooming; a hairdressing input check box (□) for inputting presence/absence of hairdressing; an oral care input check box (□) for inputting presence/absence of oral care; a wash-up input check box (□) for inputting presence or absence of wash-up; a bed-leaving input check box (□) for inputting presence/absence of bed-leaving; a skin-wiping input check box (□) for inputting presence/absence of skin-wiping; and others input check box (□) for inputting presence or absence of others.

In response to input operation (for example, tapping) on each of the areas 641b-2 and 641b-3 in the care record input area 641b of the morning care recording format screen 64b illustrated in Fig. 7, a data input subwindow in accordance with attribute of data (for example, text data, time data, etc.) to be input into the areas 641b-2 and 641b-3 opens to be superimposed on the morning care recording format screen 64b similarly to the meal care recording format screen 64a.

A bowl movement care recording format screen 64c in Fig. 8 is a screen for displaying a recording format for bowl movement care, and is a screen for inputting the content of the implemented bowl movement care as a care record. For example, as illustrated in Fig. 8, the bowl movement care recording format screen 64c includes: a menu bar area 511; a target person name display area 631; and a care record input area 641c that displays input items as to be input as content of care for inputting the content of care according to the input item as a care record; a sharing check box (□) 642; and a "transmit" button 643.

In the recording format for bowl movement care, for example, in the present embodiment, the input items include urination, a bowl movement amount, bowl movement quality, the memo, and the care implementation time. In order to input these, the care record input area 641c includes: a urination input area 641c-1 for inputting the presence/absence of urination; a bowl movement amount input area 641c-2 for inputting the bowl movement amount; a bowl movement quality input area 641c-3 for inputting the form of bowl movement; a free description input area 641c-4 for inputting a memo; and a care implementation time input area 641c-5 for inputting the time when the monitored person Ob received care.

In response to input operation (for example, tapping) on each of the areas 641c-1 to 641c-5 in the care record input area 641c of the bowl movement care recording format screen 64c illustrated in Fig. 8, a data input subwindow in accordance with attribute of data (for example, text data, time data, etc.) to be input into the area 641c-1 to 641c-5 is supposed to be superimposed to open over the bowl movement care recording format screen 64c similarly to the meal care recording format screen 64a.

The format information as described above is stored in the format information storage unit 234 in association with one of a plurality of different recording formats according to the type of care with respect to a predetermined status parameter. The status parameter is a parameter that represents a predetermined status at a transmission source that has requested a recording format. As described above, the recording format often depends on the position (location), the monitoring person (user), the time, or the like. For example, the recording format for bowl movement care depends on the position (location) since it is often used near the toilet. Furthermore, for example, in a case where the person in charge of the monitored person Ob-1 is a caregiver NS-1, the recording format for the monitored person depends on the monitoring person. In another case, for example, the recording format for meal care depends on the time, since it is often used around the meal time.

Accordingly, in the present embodiment, the status parameter is at least one of position information indicating the position of the transmission source, the terminal device SP of the transmission source, monitoring person information indicating a monitoring person who handles the terminal devices SP and TA, or requested time of the recording format. In the present embodiment, the request for the recording format is received by a format request communication signal being a communication signal containing a status parameter representing a predetermined status at the transmission source and a command for requesting the recording format. Accordingly, the recording format request time is the reception time of the format request communication signal. In the present embodiment, the status parameter includes the position information, the monitoring person information, and the reception time. Such format information is stored in the form of a table in the format information storage unit 234 in the present embodiment, for example.

More specifically, for example, as illustrated in Fig. 9, a format information table FT for registering format information includes: a status parameter field 2341 for registering status parameters; and a recording format field 2342 for registering a recording format corresponding to the status parameter registered in the status parameter field 2341. Each of the status parameters has a record.

As described above, the status parameter in the present embodiment includes the position information, the monitoring person information, and the reception time. Accordingly, the status parameter field 2341 includes: a location sub-field 23411 for registering the position information; a monitoring person sub-field 23412 for registering the monitoring person information; and a time sub-field 23413 for registering the reception time.

A plurality of candidates is prepared for the recording format corresponding to the status parameter. For example, since two candidates are prepared in the present embodiment, the recording format field 2342 includes a first candidate format sub-field 23421 for registering a recording format of the first candidate; and a second candidate format sub-fields 23422 for registering a recording format of the second candidate. Although the first and second candidate format sub-fields 23421 and 23422 may register information of the recording format itself, a file name (for example, recording format name, etc.) in an electronic file of the recording format is registered in the present embodiment. The electronic file of the recording format is described in a markup language such as Hyper Text Markup Language (HTML) or Extensible Markup Language (XML), for example, and is stored in the format information storage unit 234.

The care record information storage unit 235 stores the care record information. The name of the monitored person as a care recipient, the content of the care record, and an image in a case where the image is photographed in association with the care by the photographing function of the mobile terminal device TA are stored as the care record information in the care record information storage unit 235 in association with one another. The care record information is stored in the care record information storage unit 235 in the form of a table in the present embodiment.

As illustrated in Fig. 10, a server side care record information table (SV care record information table) NR-SV for registering the care record information includes: a monitored person name field 2341 for registering the monitored person name; a care content field 2342 for registering the content of care; a photographing time field 2343 for registering the time when the image is photographed; and a still image field 2344 for registering a still image in a case where the photographed image is a still image; and a moving image field 2345 for registering a moving image in a case where the photographed image is a moving image. Each of the cares has a record.

For example, the care content field 2342 has registered a file name of the recording format screen 64 (or the meal care recording format screen 64a in the example illustrated in Fig. 6) having description of the content of care, the corresponding event name, or the like. For example, each of the still image field 2344 and the moving image field 2345 may register image data of a still image and a moving image, respectively, for example, and may register a file name of image data of a still image or a moving image, respectively.

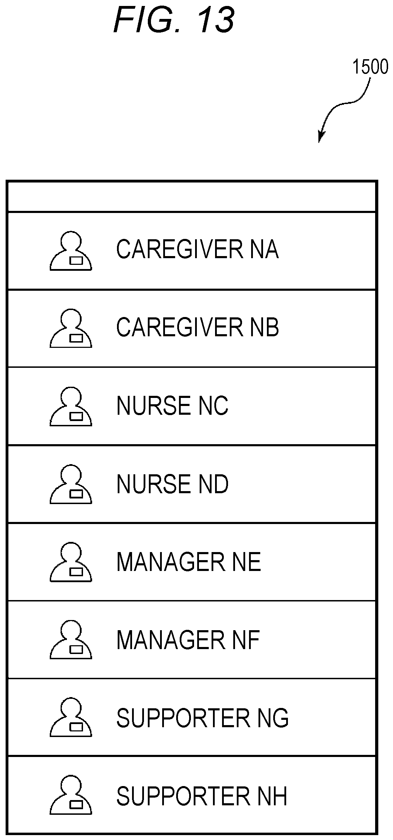

The user information storage unit 236 stores user information. In the present embodiment, the user information is information of each of the monitoring persons (users) holding the mobile terminal device TA. Such user information is stored in the user information storage unit 236 in the form of a table in the present embodiment. From this user information, a user list selection screen 1500 (Fig. 13) described below is generated.

The SV control processing unit 22 is a circuit that controls individual units of the management server device SV according to the functions of the individual units. When receiving the notification of the predetermined event from the sensor device SU, the SV control processing unit 22 manages the monitoring information related to the monitoring of the monitored person Ob and notifies the predetermined terminal devices SP and TA of the predetermined event, then, supplies data corresponding to the client's request to the client, and manages the entire assistance system MS.

Subsequently, in the present embodiment, when request for the recording format is received from the terminal devices SP and TA, the SV control processing unit 22 returns the recording format based on a status parameter in the terminal device SP or TA as a transmission source, to the terminal device SP or TA being the transmission source. After reception of a care record using the recording format, the SV control processing unit 22 stores the care record. The SV control processing unit 22 includes, for example, a central processing unit (CPU) and its peripheral circuits.

The SV control processing unit 22 executes the control processing program to functionally implement: a server side control unit (SV control unit) 221, a server side monitoring processing unit (SV monitoring processing unit) 222; a format distribution processing unit 223; a server side care record processing unit (SV care record processing unit) 224; and a login authentication processing unit 225. The login authentication processing unit 225 will be described below.

The SV control unit 221 controls individual units of the management server device SV in accordance with the functions of the individual units, and performs overall control of the management server device SV.

After reception of a notification of the predetermined event from the sensor device SU, the SV monitoring processing unit 222 manages monitoring information related to monitoring of the monitored person Ob and reports the predetermined event to the predetermined terminal devices SP and TA. More specifically, after reception of the first event notification communication signal from the sensor device SU, the SV monitoring processing unit 222 stores monitoring information contained in the received first event notification communication signal and related to monitoring of the monitored person Ob, into the SV monitoring information storage unit 231.

Subsequently, the SV monitoring processing unit 222 selects a notification destination corresponding to the sensor device SU that has transmitted the received first event notification communication signal from among the notification destination correspondence relationships stored in the inter-device information storage unit 232, and then transmits a second event notification communication signal to the selected terminal devices SP and TA. The selection processing is performed on the basis of the sensor ID corresponding to the sensor device SU that has transmitted the received first event notification communication signal.

In a case where the event information contained in the first event notification communication signal is the predetermined behavior (one or more of sitting-up, bed-leaving, falling and tumbling), the second event notification communication signal would contain a sensor ID contained in the first event notification communication signal, event information and a target image, and a communication address corresponding to the sensor device SU having the sensor ID accommodated in the first event notification communication signal as a download destination of a moving image. The communication address is selected from the communication address correspondence relationship on the basis of the sensor ID corresponding to the sensor device SU that has transmitted the received first event notification communication signal. In a case where the event information contained in the first event notification communication signal is the nurse call, the second event notification communication signal would contain the sensor ID and event information (nurse call) contained in the first event notification communication signal.

When receiving a request for a recording format from the terminal device SP or TA, the format distribution processing unit 223 returns the recording format based on the status parameter in the terminal device SP or TA as the transmission source, to the terminal device SP or TA as the transmission source. Subsequently, in the present embodiment, the format distribution processing unit 223 counts the date and time.

More specifically, when receiving by the SV communication IF unit 21, of a first format request communication signal being a communication signal that contains a first command that requests for a status parameter representing a predetermined status on the transmission source and the recording format, the format distribution processing unit 223 selects a recording format from among a plurality of recording formats stored in the format information storage unit 234 on the basis of the status parameter contained in the received format request communication signal and transmits a first format reply communication signal being a communication signal containing the selected recording format, to the transmission source via the SV communication IF unit 21.

Subsequently, when receiving by the SV communication IF unit 21, of a second format request communication signal being a communication signal containing a second command that requests for a second recording format different from the recording format contained in the first format reply communication signal, from the transmission source that has transmitted the first format reply communication signal, the format distribution processing unit 223 selects a second recording format different from the recording format selected for the first format reply communication signal from among the plurality of recording formats stored in the format information storage unit 234 on the basis of the status parameter contained in the first format request communication signal received from the transmission source and attributed to the transmission of the first format reply communication signal, and transmits a second format reply communication signal being the communication signal containing the selected second recording format to the transmission source by the SV communication IF unit 21.

More specifically, when receiving the first format request communication signal from the terminal device SP or TA by the SV communication IF unit 21, the format distribution processing unit 223 selects a status parameter contained in the received first format request communication signal and selects a record to be registered to the status parameter field 2341 from the format information table FT stored in the format information storage unit 234, and takes out a recording format (file name of an electronic file of the recording format in the present embodiment) recorded in a first candidate format sub-field 23421 in the recording format field 2342 of the selected record, and transmits the first format reply communication signal containing the extracted recording format (electronic file of the recording format corresponding to the file name, in the present embodiment) to the transmission source by the SV communication IF unit 21.

Subsequently, when receiving the second format request communication signal by the SV communication IF unit 21 from the transmission source that has transmitted the first format reply communication signal, the format distribution processing unit 223 selects the status parameter contained in the first format request communication signal received from the transmission source and attributed to transmission of the first format reply communication signal and selects a record to be registered to the status parameter field 2341 from the format information table FT stored in the format information storage unit 234, and extracts a recording format (file name of an electronic file of the recording format in the present embodiment) registered to a second candidate format sub-field 23422 in the recording format field 2342 on the selected record, and transmits the second format reply communication signal containing the extracted recording format (electronic file of the recording format corresponding to the file name, in the present embodiment) to the transmission source by the SV communication IF unit 21.

When receiving a care record using the recording format from the terminal devices SP and TA, the SV care record processing unit 224 stores the care record in the care record information storage unit 235.

As indicated by a broken line in Fig. 2, the management server device SV may further include: a server side input unit (SV input unit) 24 connected to the SV control processing unit 22 and used for inputting various commands, various data, or the like; a server side output unit (SV output unit) 25 for outputting various commands and various data input from the SV input unit 24 and monitoring information regarding monitoring of the monitored person Ob or the like; and a server side interface unit (SVIF unit) 26 for performing data input and output with external devices.

Such a management server device SV can be constituted by, for example, a computer with a communication function.

In the present embodiment, the fixed terminal device SP is installed at the nurse station ST and used by the staff of the nurse station ST. The fixed terminal device SP includes a communication function of communicating with other devices SU, SV, and TA via the network NW, a display function of displaying predetermined information, and an input function of inputting predetermined instructions and data, and functions as a user interface (UI) of the assistance system MS by performing operation such as inputting predetermined instructions and data to be provided to the management server device SV and the mobile terminal device TA and displaying monitoring information obtained by the sensor device SU. Such a fixed terminal device SP can be constituted by, for example, a computer with a communication function. The fixed terminal device SP will be described in detail below.

The mobile terminal device TA includes a communication function of communicating with other devices SV, SP, and SU via the network NW, a display function of displaying predetermined information, an input function of inputting predetermined instructions and data, and a call function of performing voice calling, so as to be used for inputting predetermined instructions and data to be provided to the management server device SV and the sensor device SU, for displaying the monitoring information obtained by the sensor device SU by notification from the management server device SV, and for responding to a nurse call, giving a voice message, or the like, by voice calling with the sensor device SU. The mobile terminal device TA is carried, for example, by a monitoring person (user) such as a nurse or a caregiver.

As illustrated in Fig. 11, for example, such a mobile terminal device TA in the present embodiment includes: a terminal side communication interface unit (TA communication IF unit) 31; a terminal side control processing unit (TA control processing unit) 32; a terminal side storage unit (TA storage unit) 33; a terminal side sound input/output unit (TA sound input/output unit) 34; a terminal side input unit (TA input unit) 35; a terminal side display unit (TA display unit) 36; a terminal side interface unit (TAIF unit) 37; and a camera 38.

The TA sound input/output unit 34 is a circuit connected to the TA control processing unit 32 for obtaining an external sound and inputting the obtained sound to the mobile terminal device TA, as well as generating a sound corresponding to an electrical signal representing the sound under the control of the TA control processing unit 32 and outputting the generated sound. For example, the TA sound input/output unit 34 includes: a speaker or the like that converts an audio electrical signal (sound data) into an audio mechanical vibration signal (acoustic signal); and a microphone that converts the mechanical vibration signal of an audible area sound into an electrical signal. The TA sound input/output unit 34 outputs an electrical signal representing an external sound to the TA control processing unit 32, as well as converting an electrical signal input from the TA control processing unit 32 into an audio mechanical vibration signal and outputting the signal.

The TA input unit 35 (corresponding to an example of the input unit) is a circuit that is connected to the TA control processing unit 32 and receives predetermined operation and inputs the operation to the mobile terminal device TA, for example. An example of this unit is a plurality of input switches each of which assigned with a predetermined function. Examples of the predetermined operation include various types of operation necessary for monitoring, such as: input operation of a user name and a passcode for login; request operation and finish operation for voice calling: request operation and finish operation for live moving image; input operation of intention ("Action") of taking action such as lifesaving, nursing, caregiving and assistance to the monitored person Ob involved in the notified event; and input operation of a care record using a predetermined recording format. The camera 38 (corresponding to an example of an imaging unit) includes an imaging element, a shutter, or the like, and has a function of photographing a still image or a moving image.

The TA display unit 36 (corresponding to an example of the display unit) is a circuit connected to the TA control processing unit 32, and performs display, under the control of the TA control processing unit 32, of predetermined operation content input from the TA input unit 35, the monitoring information related to the monitoring of the monitored person Ob monitored by the assistance system MS, a recording format for inputting a care record, and images or the like photographed by the camera 38. An example of the TA display unit 36 is a display device such as a liquid crystal display (LCD) or an organic EL display. As described above, examples of the monitoring information include: event information representing the predetermined event (type of the predetermined behavior detected by the sensor device SU and the nurse call received by the nurse call device (NC); images (still images and moving images) of the monitored person Ob, and the time of reception of the notification.

In the present embodiment, the TA input unit 35 and the TA display unit 36 are implemented as a touch panel. In this case, the TA input unit 35 is a resistive film-type or a capacitive-type position input device that detects and inputs operation positions, for example. This touch panel includes a position input device provided on a display surface of the TA display unit 36. The TA display unit 36 displays one or more input content candidates that can be input to the TA display unit 36. For example, in response to a touch by a user (monitoring person) such as a nurse or a caregiver on a display position where the input content desired to be input is displayed, the position is detected by the position input device, and the display content displayed at the detected position is to be input into the mobile terminal device TA as the user's operation input content.

The TAIF unit 37 is a circuit connected to the TA control processing unit 32 and configured to perform data input/output with an external device under the control of the TA control processing unit 32. Examples of this unit include an interface circuit using a Bluetooth (registered trademark) standard, an interface circuit that performs infrared communication such as IrDA standard, and an interface circuit using USB standard.

The TA communication IF unit 31 is a communication circuit that is connected to the TA control processing unit 32 and configured to perform communication under the control of the TA control processing unit 32. The TA communication IF unit 31 includes, for example, a communication interface circuit conforming to the same standard as that of the SV communication IF unit 21, such as the IEEE 802.11 standard.

The TA storage unit 33 is a circuit connected to the TA control processing unit 32 and configured to store various predetermined programs and various predetermined data under the control of the TA control processing unit 32. Examples of the various predetermined programs include control processing programs such as: a TA control program for controlling individual portions of the mobile terminal device TA in accordance with the function of individual portions; a TA monitoring processing program that executes predetermined information processing related to monitoring of the monitored person Ob; a call processing program that makes voice calling with the sensor device SU by using the TA sound input/output unit 34 or the like; a TA streaming processing program that receives distribution of a moving image from the sensor device SU and that displays the distributed moving image on the TA display unit 36 in streaming reproduction; a TA care recording processing program that executes care record input processing using a predetermined recording format; a login processing program that executes login processing; and a photographing processing program that executes photographing processing using the camera 38.

The various predetermined data includes data necessary for execution of each of programs, such as the terminal ID of its own device, the monitoring information of the monitored person Ob, the sensor information regarding the sensor device SU, and the screen information displayed on the TA display unit 36. The TA storage unit 33 includes a ROM and an EEPROM, for example. The TA storage unit 33 includes, for example, a RAM serving as a working memory of the TA control processing unit 32 for storing data or the like generated during execution of the predetermined program.

In order to individually store the monitoring information, the sensor information, and the screen information, the TA storage unit 33 functionally includes: a terminal side monitoring information storage unit (TA monitoring information storage unit) 331; a terminal side sensor information storage unit (TA sensor information storage unit) 332; and a screen information storage unit 333. The TA storage unit 33 further includes an image memory 334 that temporarily saves an image photographed by the camera 38.

The TA monitoring information storage unit 331 stores the monitoring information. In the present embodiment, the TA monitoring information storage unit 331 stores, as the monitoring information: a sensor ID and event information (sitting-up, bed-leaving, falling, and tumbling, and a nurse call, in the present embodiment) contained in the second event notification communication signal received from the management server device SV; a communication address of the sensor device SU as a download destination of images and the moving images; the reception time of the second event notification communication signal; and the presence or absence of the handling, or the like, in association with one another. More specifically, as illustrated in Fig. 3, the TA monitoring information storage unit 331 stores the monitoring information to a terminal side monitoring information table (TA monitoring information table) MT-TA similar to the SV monitoring information table MT-SV.

The TA sensor information storage unit 332 stores the sensor information. The TA sensor information storage unit 332 stores the sensor ID, the installation location, the monitored person name, and remarks in association with one another. More specifically, as illustrated in Fig. 5, the TA sensor information storage unit 332 stores the sensor information in a terminal side sensor information table (TA sensor information table) ST-TA similar to the SV sensor information table ST-SV.

The screen information storage unit 333 stores the screen information. The screen information storage unit 333 preliminarily stores electronic files such as a login screen 1400, a standby screen 51, a monitoring information screen 52, and a nurse call reception screen 53, which are relatively frequently used and described below, for example, as well as storing electronic files such as the predetermined recording format screen 64 received from the management server device SV.

The TA control processing unit 32 is a circuit that controls individual units of the mobile terminal device TA according to the function of the individual units, receives and displays the monitoring information of the monitored person Ob, and responds to a nurse call or gives a voice message. In the present embodiment, the TA control processing unit 32 executes a care record input processing using a predetermined recording format. The TA control processing unit 32 includes, for example, a CPU and its peripheral circuits.

The TA control processing unit 32 executes a control processing program to functionally implement: a terminal side control unit (TA control unit) 321; a terminal side monitoring processing unit (TA monitoring processing unit) 322; a call processing unit 323; a terminal side streaming processing unit (TA streaming processing unit) 324; a terminal side care recording processing unit (TA care record processing unit) 325; a login processing unit 326; and a photographing processing unit 327. The login processing unit 326 and the photographing processing unit 327 will be described below.

The TA control unit 321 controls individual units of the mobile terminal device TA according to the function of the individual units, and performs the entire control of the mobile terminal device TA.

The TA monitoring processing unit 322 executes predetermined information processing related to monitoring of the monitored person Ob. More specifically, when receiving the second event notification communication signal transmitted by the management server device SV, attributed to the first event notification communication signal transmitted by the sensor device SU, the TA monitoring processing unit 322 stores the monitoring information of the monitored person Ob into the TA monitoring information storage unit 331 on the basis of the information (data) contained in the received second event notification communication signal. The TA monitoring processing unit 322 displays a screen corresponding to each of pieces of information contained in the received second event notification communication signal, on the TA display unit 36. When receiving predetermined input operation from the TA input unit 35, the TA monitoring processing unit 322 executes predetermined processing according to the input operation.

The call processing unit 323 performs voice calling with the sensor device SU by using the TA sound input/output unit 34 or the like. More specifically, for example, the call processing unit 323 uses the TA sound input/output unit 34 or the like and performs voice calling via Voice over Internet Protocol (VoIP) with the sensor device SU as a transmission source that has transmits the first event notification communication signal that caused the transmission of the second event notification communication signal, to the management server device SV.

The TA streaming processing unit 324 receives distribution of a moving image (for example, a live moving image) from the sensor device SU, and displays the distributed moving image on the TA display unit 36 in streaming reproduction.

The TA care record processing unit 325 executes a care record input processing using a predetermined recording format. More specifically, the TA care record processing unit 325 executes care record input processing in two modes, namely, first and second modes. In the first mode, the TA care record processing unit 325 sequentially executes processing of: target person selection processing of selecting a target person for care recording; format selection processing of selecting a recording format; format request display processing of requesting a recording format to the management server device SV and displaying the format on the TA display unit 36; and recording processing of receiving the care record input using the recording format by the TA input unit 35 and transmitting the input care record to the management server device SV.

In the second mode, in response to the reception of a recording format request at the TA input unit 35, the TA care record processing unit 325 sequentially executes processing of: acquisition processing of obtaining a status parameter of an own device (location information of the own device in the present embodiment); first format request display processing of transmitting a first format request communication signal containing the terminal ID of the own device, the status parameter of the own device, and the first command requesting a recording format to the management server device SV and displaying a recording format (first candidate recording format) contained in a returned first format reply communication signal on the TA display unit 36; second format request display processing of transmitting a second format request communication signal containing the terminal ID of the own device, and a second command requesting a second recording format different from the recording format contained in the first format reply communication signal, to the management server device SV as necessary, and displaying a recording format (second candidate recording format) contained in a returned second format reply communication signal on the TA display unit 36; and recording processing of receiving, at the TA input unit 35, an input of a care record using either the first candidate recording format or the second candidate recording format at the TA input unit and transmitting the input care record to the management server device SV.

Such a mobile terminal device TA can be implemented by a portable communication terminal device such as a tablet type computer, a smartphone, or a cellular phone.

### (Operation)

Next, operation of the present embodiment will be described. In the assistance system MS with the above configuration, after power is turned on, each of the devices SU, SV, SP, and TA executes initialization of necessary portions and starts its operation. The management server device SV executes the control processing program to allow the SV control processing unit 22 to functionally form the SV control unit 221, the SV monitoring processing unit 222, the format distribution processing unit 223, the SV care record processing unit 224, and the login authentication processing unit 225. The mobile terminal device TA executes the control processing program to allow the TA control processing unit 32 to functionally form the TA control unit 321, the TA monitoring processing unit 322, the call processing unit 323, the TA streaming processing unit 324, and the TA care record processing unit 325, the login processing unit 326, and the photographing processing unit 327.

### (Login)

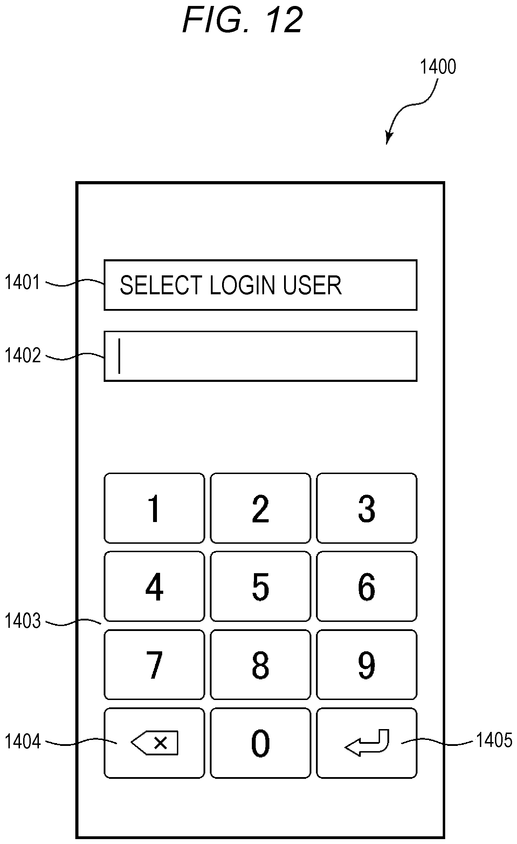

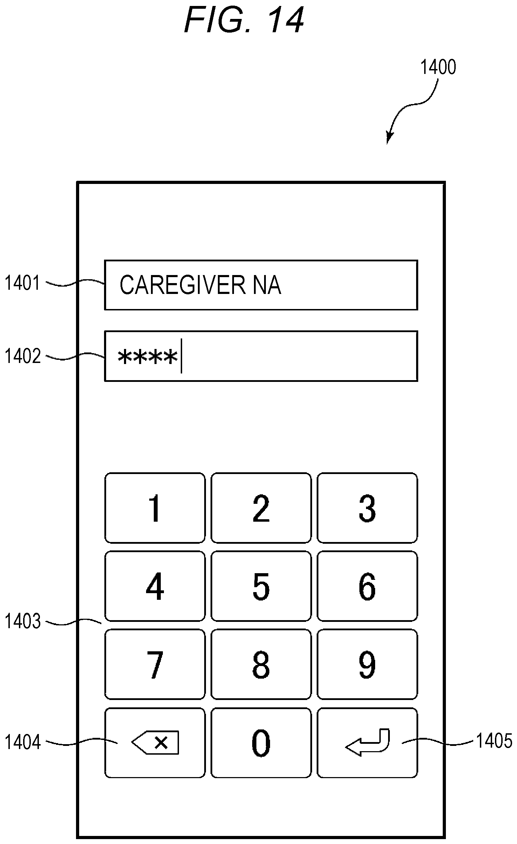

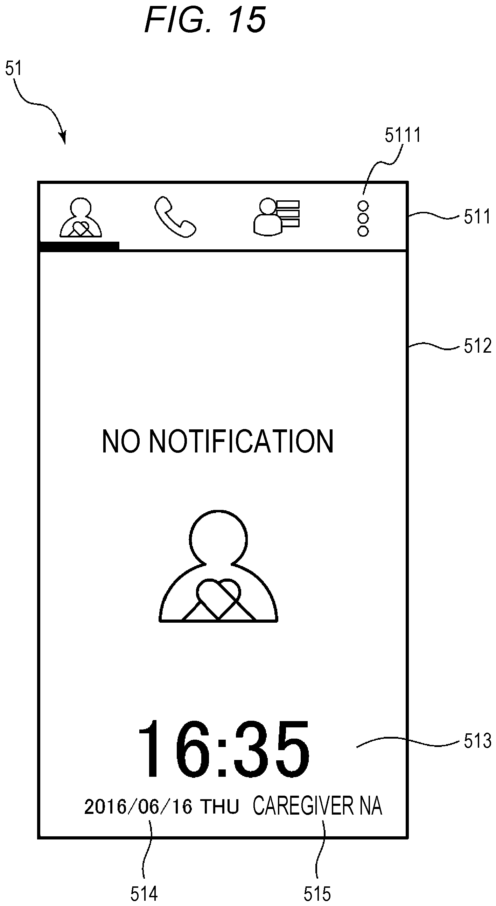

Fig. 12 is a view illustrating an example of a login screen displayed on the mobile terminal device TA. Fig. 13 is a view illustrating an example of a user list selection screen displayed on the mobile terminal device TA. Fig. 14 is a view illustrating an example of a passcode input screen displayed on the mobile terminal device TA. Fig. 15 is a view illustrating an example of a standby screen displayed on the mobile terminal device TA. Login operation of the mobile terminal device TA will be described with reference to Figs. 11 to 15.

When the power of the mobile terminal device TA is turned on, the operating system is started and thereafter, an assistance application in which the mobile terminal device TA operates as a component of the assistance system MS is automatically started. The login processing unit 326 displays the login screen 1400 illustrated in Fig. 12 on the TA display unit 36. The mobile terminal device TA may be configured to start the assistance application and display the login screen 1400 by performing an input operation (tapping) on an icon for the assistance system MS displayed on the screen after the operating system is started. As illustrated in Fig. 12, the login screen 1400 includes a user selection field 1401 displayed as "select login user ", a passcode input field 1402, a numeric keypad 1403, an erase button 1404, and a line-feed button 1405.

When input operation (tapping) is performed on the user selection field 1401, the login processing unit 326 transmits a request signal including a user list transmission request and a terminal ID to identify the mobile terminal device TA to the management server device SV by the TA communication IF unit 31. When the SV communication IF unit 21 of the management server device SV has received the request signal, the login authentication processing unit 225 reads the user information from the user information storage unit 236. The login authentication processing unit 225 transmits a response signal including the read user information to the mobile terminal device TA identified by the terminal ID by the SV communication IF unit 21. The login processing unit 326 of the mobile terminal device TA extracts a list of monitoring persons (users) from the response signal received by the TA communication IF unit 31 to generate a user list selection screen 1500, and then displays the generated screen on the TA display unit 36. As illustrated in Fig. 13, the user list selection screen 1500 lists monitoring persons (users) who hold the mobile terminal device TA registered in the management server device SV.

Performing input operation (tapping) on the user's own name on the user list selection screen 1500 generates a state in which the user's own name has been entered in the user selection field 1401 instead of "select login user" on the login screen 1400 of Fig. 12. In this state, as illustrated in Fig. 14, when the passcode is input using the numeric keypad 1403 and input operation (tapping) is performed on the line-feed button 1405, the login processing unit 326 controls the TA communication IF unit 31 to transmit an inquiry signal including the passcode and the selected the user's own name to the management server device SV to inquire whether the passcode is correct. After the SV communication IF unit 21 of the management server device SV has received the above passcode, the login authentication processing unit 225 compares the received passcode with a previously held passcode. When the comparison results match, the login authentication processing unit 225 determines that the login authentication is normally completed.

When the login authentication is determined to be completed normally, the SV monitoring processing unit 222 of the management server device SV stores the above-described terminal ID and the monitoring person's name (monitoring person's information) in association with each other in the SV storage unit 23. The SV communication IF unit 21 transmits a response signal including a signal indicating normal completion of the login authentication created by the login authentication processing unit 225, to the logged-in mobile terminal device TA.

When a response signal including a signal indicating normal completion of the login authentication has been received, the TA monitoring processing unit 322 of the mobile terminal device TA displays a standby screen 51 (Fig. 15) that waits for a communication signal addressed to the own device, on the TA display unit 36.

For example, as illustrated in Fig. 15, this standby screen 51 includes a menu bar area 511 that displays a menu bar; a standby main area 512 that displays a message (for example, "No notification") and an icon indicating that the device is in a standby state; a time area 513 that displays the current time; a year/month/date/day area 514 for displaying the current year/month/date and day of the week; and a user name area 515 that displays the name of the user who is currently in a log-in state to the mobile terminal device TA. The menu bar area 511 includes a sub-menu button 5111.

### (Care related image recording operation)

From the state where the standby screen 51 illustrated in Fig. 15 is displayed on the TA display unit 36, care related image recording operation described below is started. A care related image is an image photographed by the monitoring person (user) using the camera 38 and related to the care content given by the monitoring person for the monitored person Ob. Hereinafter, first to fourth examples of the care related image recording operation will be described.

### (First example of care related image recording operation)

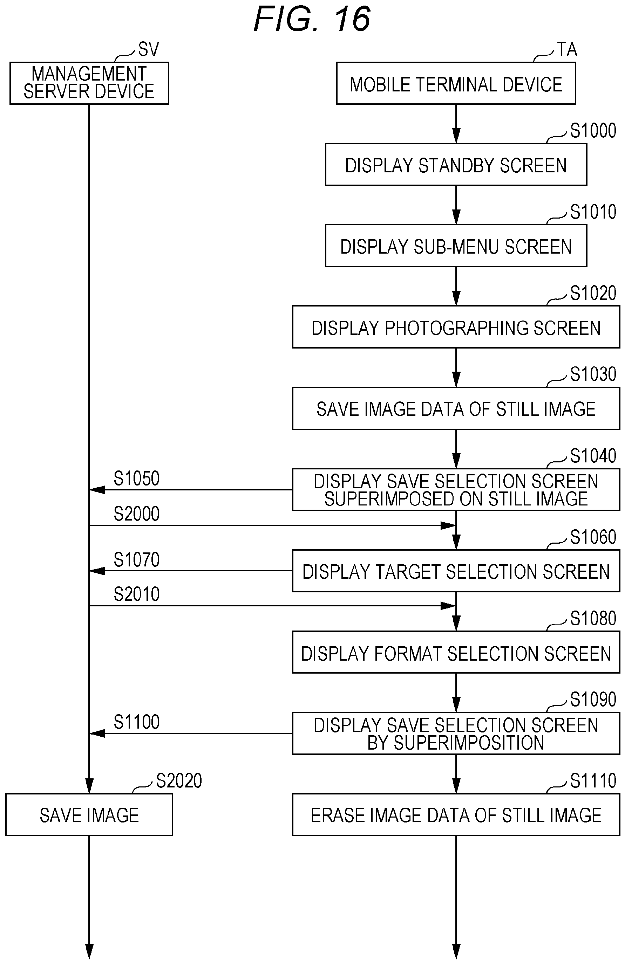

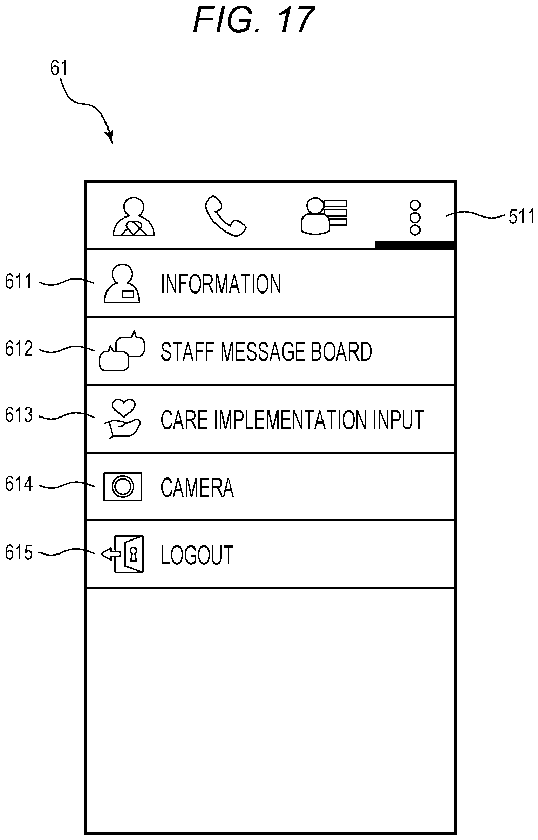

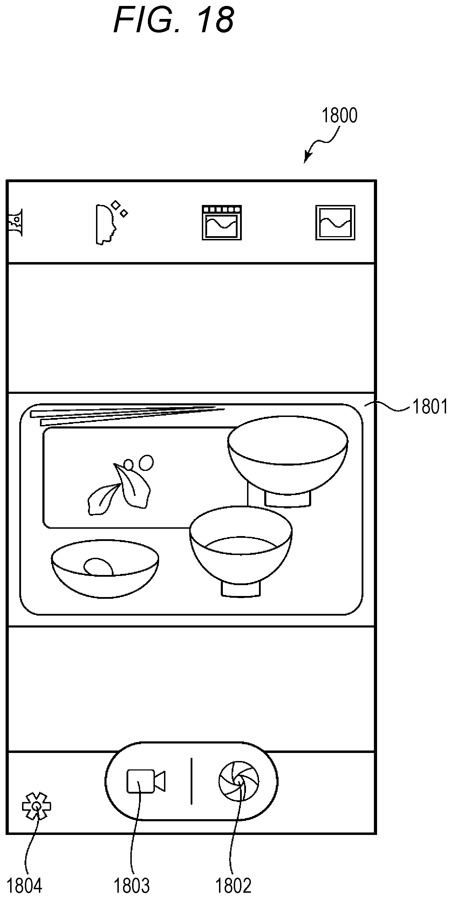

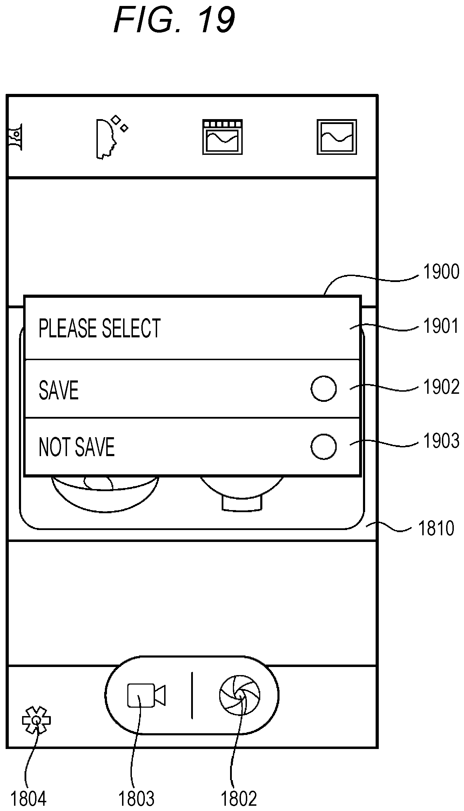

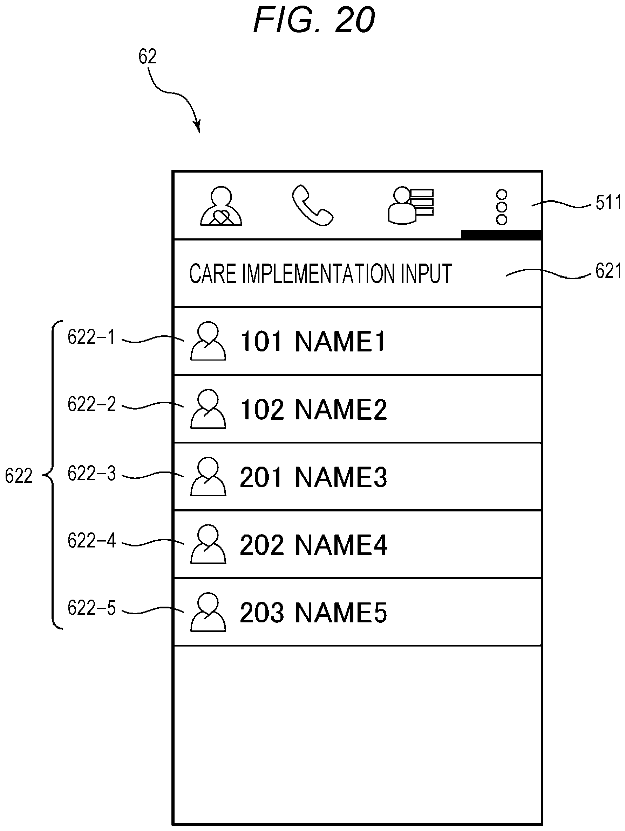

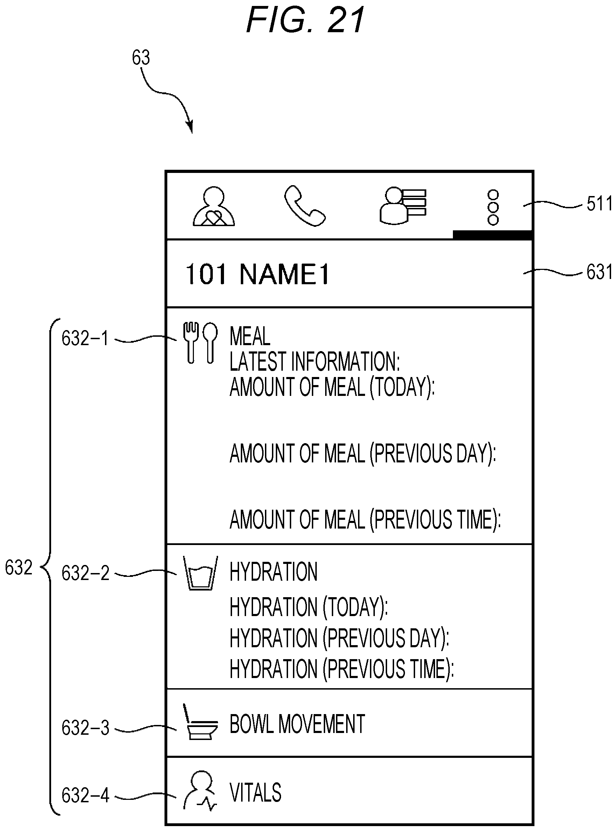

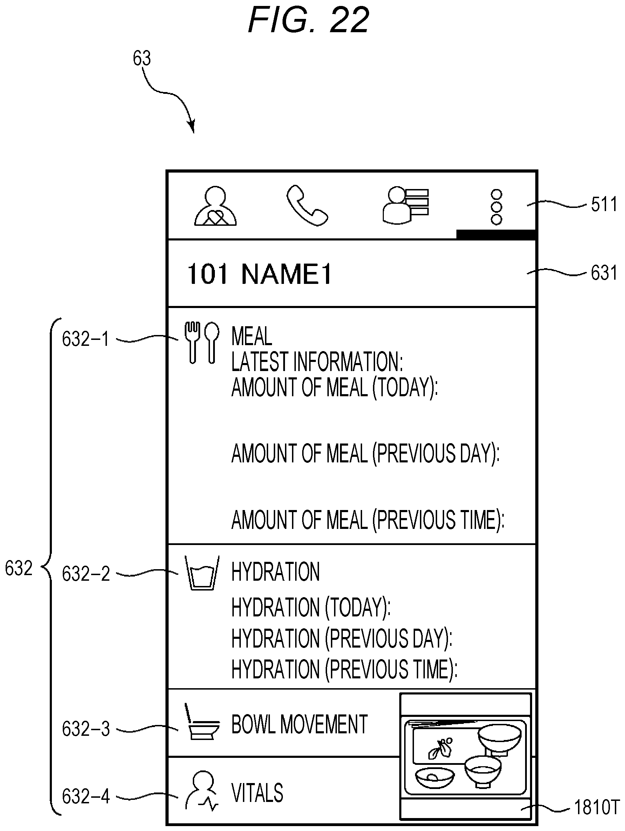

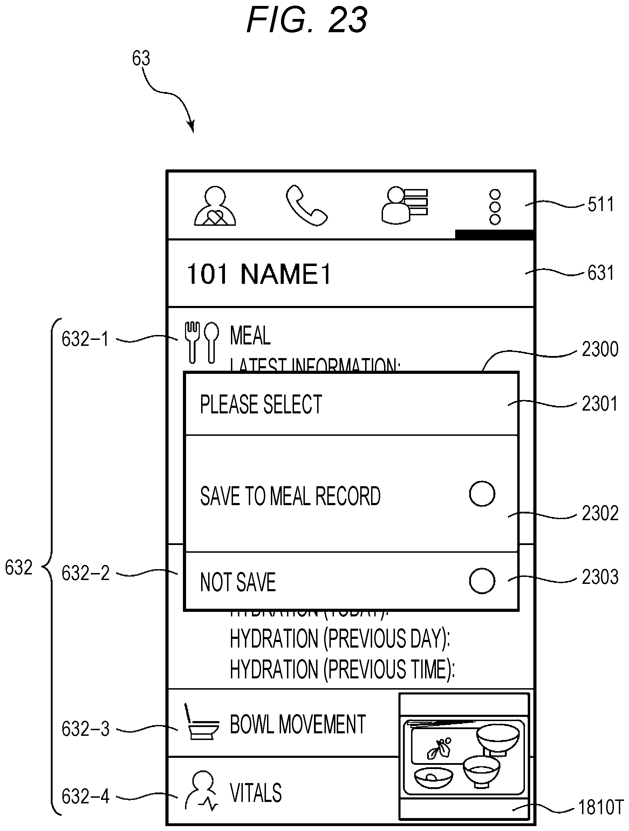

Fig. 16 is a sequence diagram schematically illustrating a first example of care related image recording operation. Fig. 17 is a view illustrating an example of a sub-menu screen 61 displayed on the mobile terminal device TA. Fig. 18 is a view illustrating an example of a photographing screen 1800 displayed on the mobile terminal device TA. Fig. 19 is a view illustrating an example of a save selection screen 1900 displayed on the mobile terminal device TA. Fig. 20 is a view illustrating an example of a target person selection screen 62 displayed on the mobile terminal device TA. Fig. 21 is a view illustrating an example of a format selection screen 63 displayed on the mobile terminal device TA. Fig. 22 is a view illustrating an example in which a thumbnail image is displayed on the format selection screen 63. Fig. 23 is a view illustrating an example of a save selection screen 2200 displayed on the mobile terminal device TA.

When the login authentication is normally completed as described above in step S1000 in Fig. 16 (corresponding to an example of the standby screen display step), the TA display unit 36 of the mobile terminal device TA displays the standby screen 51 (Fig. 15). When input operation (tapping) using the TA input unit 35 is performed on the sub-menu button 5111 of the menu bar area 511 in the standby screen 51 in step S1010, the TA care record processing unit 325 of the mobile terminal device TA displays the sub-menu screen 61 illustrated in Fig. 17 for example, on the TA display unit 36.

The sub-menu button 5111 of the standby screen 51 (Fig. 15) is a button for inputting an instruction to display the sub-menu screen 61, into the mobile terminal device TA. The sub-menu screen 61 is a screen that displays selectable sub-menus to select a sub-menu. For example, as illustrated in Fig. 17, the sub-menu screen 61 includes, as sub-menus, an "information" button 611, a "staff message board" button 612, a "care implementation input" button 613, a "camera" button 614, and a "logout" button 615.

The "information" button 611 is a button for inputting an instruction to display predetermined information such as a login name (caregiver name) and a terminal ID, into the mobile terminal device TA. The "staff message board" button 612 is a button used to input an instruction to display a message registered in an electronic message board recorded by the monitoring person (user) from the terminal device SP or TA on the management server device SV, into the mobile terminal device TA. The "care implementation input" button 613 is a button used to input an instruction to record a care record on the management server device SV by the monitoring person from the mobile terminal device TA, into the mobile terminal device TA. The "logout" button 615 is a button used for inputting an instruction to logout, into the mobile terminal device TA. The "camera" button 614 is a button used for inputting an instruction to activating a camera application that controls the camera 38, into the mobile terminal device TA.

In step S1020 of Fig. 16, when input operation (tapping) using the TA input unit 35 is performed on the "camera" button 614, the camera application is activated and the mobile terminal device TA enters a photographing mode to be implemented by the photographing processing unit 327. In this manner, during the activation of the assistance application, the camera application can be activated by displaying the sub-menu screen 61. When the mobile terminal device TA enters the photographing mode, the photographing processing unit 327 displays the photographing screen 1800 on the TA display unit 36. For example, as illustrated in Fig. 18, the photographing screen 1800 includes an image display area 1801 provided at the center of the TA display unit 36, and a shutter button 1802, a video button 1803, and a setting button 1804, provided at a lower portion of the TA display unit 36.

The image display area 1801 is an area that displays an image being photographed. The shutter button 1802 is a button for instructing the camera 38 to photograph a still image. The video button 1803 is a button for instructing the camera 38 to start and stop photographing a moving image. The setting button 1804 is a button for instructing the camera 38 to set a photographing mode (for example, landscape, proximity, indoor). The image display area 1801 in Fig. 18 displays a tray on which the dishes having some food left are placed, which indicates that the example in Fig. 18 is an image after a meal.

When input operation (taping) is performed on the shutter button 1802 in step S1030 (corresponding to an example of an imaging step, corresponding to an example of a temporary saving step), the photographing of the still image is finished, and then, the photographing processing unit 327 temporarily saves the image data of the still image into the image memory 334 of the TA storage unit 33. In step S1040 (corresponding to an example of the save selection screen display step), the photographing processing unit 327 displays the still image 1810 saved in the image memory 334 on the TA display unit 36, and together with this, displays the save selection screen 1900 over the still image 1810, as illustrated in Fig. 19, for example. The save selection screen 1900 includes a message display area 1901 describing "Please select", a "Save" button 1902, and a "Not save" button 1903. When input operation is performed on the "Not save" button 1903, the photographed still image is discarded, the processing returns to the photographing mode in step S1020, and the photographing screen 1800 is displayed on the TA display unit 36.

When input operation is performed on the "Save" button 1902, the mobile terminal device TA shifts to the care record input mode in step S1050, and the TA care record processing unit 325 controls the TA communication IF unit 31 to notify the management server device SV of the input operation on "Save" button 1902 and of the shift to the care record input mode.

In response to this notification, in step S2000, the SV care record processing unit 224 of the management server device SV creates the target person selection screen 62, and transmits the created target person selection screen 62 to the mobile terminal device TA. In step S1060 (corresponding to an example of the target person selection screen display step), the TA care record processing unit 325 of the mobile terminal device TA displays the target person selection screen 62 transmitted from the management server device SV on the TA display unit 36.

The target person selection screen 62 is a screen used for displaying a selectable target person and selecting a care record target person of corresponding to the photographed image. For example, as illustrated in Fig. 20, the target person selection screen 62 includes a menu bar area 511, a sub-menu name display area 621 for displaying a sub-menu name, and a target person display area 622 (622-1 to 622-5) that displays one or more selectable target persons in the form of list.

The target person selection screen 62 is created as follows by the SV care record processing unit 224 of the management server device SV. In Fig. 20, the sub-menu name display area 621 displays "care implementation input" as an example in which an image is photographed during the implementation of care.

In order to display selectable target persons in the form of list in the target person display area 622, the name of the monitored person and the installation location registered in the monitored person name field 2333 and in the installation location field 2332 are extracted from individual records in the SV sensor information table ST-SV (Fig. 5) stored in the SV sensor information storage unit 233, and then, installation location and the monitored person's name, as a set, are sorted by a predetermined criterion and displayed in the form of list from top to bottom in front view of the target person selection screen 62, in the target person display area 622. The criterion for sorting can be any criterion, for example, may be sorted in the Japanese syllabary order or alphabetical order of the names of monitored persons, in the order of the room numbers of the installation locations, or the like. In the example illustrated in Fig. 20, each of sets of the installation locations and the monitored person names is sorted in the order of the room numbers of the installation locations.

In a case where the target person display area 622 is wider than the display area of the TA display unit 36 and all sets of the installation location and the monitored person's name cannot be displayed in the display area of the TA display unit 36, it would be possible, for example, to use flick input to alter the area portion of the target person display area 622 displayed on the display area of the TA display unit 36, making it possible to display a set of an installation location and a monitored person name, that is not displayed in the display area of the TA display unit 36. In addition, the individual areas 622-1 to 622-5 for displaying each of sets of the installation location and the name of the monitored person are also the target person selection buttons for inputting the target person selected by the monitoring person (user of the mobile terminal device TA) into the mobile terminal device TA.

Referring back to Fig. 16, when the input operation is performed on the area 622 (that is, the target person selection button) that displays a set of the installation location and the monitored person name by the TA input unit 35 in step S1070 (corresponding to an example of the target person transmission step) in the state where the target person selection screen 62 is displayed and the selection of the target person has been received, the TA care record processing unit 325 stores the selected target person (monitored person) in the TA storage unit 33 as a care record target person, and together with this, controls the TA communication IF unit 31 to notify the management server device SV of the selected target person.

In response to this notification, in step S2010, the format distribution processing unit 223 of the management server device SV transmits the format selection screen 63 illustrated in Fig. 21 to the mobile terminal device TA, for example. In step S1080 (corresponding to an example of the target care type selection screen display step), the TA care record processing unit 325 of the mobile terminal device TA displays the format selection screen 63 transmitted from the management server device SV on the TA display unit 36.

This format selection screen 63 (corresponding to an example of the target care type selection screen) is a screen that displays a selectable recording format prepared in advance according to the content of care in order to enable selection of the recording format. For example, as illustrated in Fig. 21, the format selection screen 63 includes: a menu bar area 511; a target person name display area 631 that displays the name of a target person selected on the target person selection screen 62; and a format display area 632 (632-1 to 632-4) that displays one or more selectable recording formats in the form of a list of the names of the recording formats.

Fig. 21 illustrates an exemplary case where input operation is performed on an area (target person selection button) 622-1 that displays "101 NAME 1" on the target person selection screen 62 of Fig. 20. Accordingly, "101 NAME 1" is displayed in the target person name display area 631.

The target person name display area 631 is also a button for inputting an instruction to display the target person selection screen 62 to the mobile terminal device TA. Therefore, the target person of the target person selection screen 62 can be changed by performing input operation on the target person name display area 631. In a case where the format display area 632 is wider than the display area of the TA display unit 36 and all the recording format names cannot be displayed in the display area of the TA display unit 36, it would be possible, for example, to use flick input to alter the area portion of the format display area 632 displayed on the display area of the TA display unit 36, making it possible to display the recording format name that is not displayed in the display area of the TA display unit 36.

The individual areas 632-1 to 632-4 that display the recording format names are also format selection buttons for inputting the recording format selected by the monitoring person to the mobile terminal device TA. In the example illustrated in Fig. 21, the format display area 632 includes: a "meal" area 632-1 that displays "meal" as a recording format name for meal care; a "hydration" area 632-2 that displays "hydration" as a recording format name for hydration care; a "bowl movement" area 632-3 that displays "bowl movement" as a recording format name for bowl movement care; and a "vital" area 632-4 that displays "vital" as a recording format name for vital check.

The "meal" area 632-1 is a button for inputting the selection of the recording format for a meal care to the mobile terminal device TA, the "hydration" area 632-2 is a button for inputting the selection of the recording format for hydration care to the mobile terminal device TA, and the "bowl movement" area 632-3 is a button for inputting the selection of the recording format for bowl movement care into the mobile terminal device TA. The "vital" area 632-4 is a button for inputting selection of a recording format for vital check to the mobile terminal device TA. In the example illustrated in Fig. 21, the content of the care record can be displayed in the individual areas 632-1 to 632-4 that display the recording format names. For example, it is possible to display the contents of the care record for today, the previous day, and the previous time.

The individual areas 632-1 to 632-4 that display the recording format names are also image save buttons for instructing saving of the photographed image in association with the recording format selected by the monitoring person.

Referring back to Fig. 16, as illustrated in Fig. 22, the TA care record processing unit 325 of the mobile terminal device TA further displays, in step S1080, a screen in which a thumbnail image 1810T of the still image 1810 (Fig. 19) is added to a lower right portion of the format selection screen 63 transmitted from the management server device SV, on the TA display unit 36.

In step S1090, for example, when input operation (tapping) is performed on the "meal" area 632-1 by the TA input unit 35, the TA care record processing unit 325 displays a save selection screen 2300 to be superimposed on the format selection screen 63, as illustrated in Fig. 23. As illustrated in Fig. 23, the save selection screen 2300 includes a message display area 2301 describing "Please select", a "Save to meal record" button 2302, and a "Not save" button 2303. When input operation is performed on "Not save" button 2303, the save selection screen 2300 is erased from the TA display unit 36, and processing returns to the display of the format selection screen 63 (Fig. 22), allowing selection of other formats.

Referring back to Fig. 16, when input operation is performed on the "Save to meal record" button 2302 in step S1100 (corresponding to an example of an image transmission step, corresponding to an example of a selected target care type transmission step), the TA care record processing unit 325 (corresponding to an example of an image transmission processing unit) controls the TA communication IF unit 31 to transmit image data of the still image 1810 saved in the image memory 334 and information indicating presence of input operation of the "Save to meal record" button 2302, to the management server device SV. In step S2020 (corresponding to an example of a save-to-device step), the SV care record processing unit 224 (an example of a save-to-device processing unit) of the management server device SV saves the transmitted image data of the still image 1810 into the care record information storage unit 235 in association with the meal record (corresponding to an example of a selected target care type) of the monitored person's name "NAME 1". In step S1110 (corresponding to an example of the erasing step), the TA care record processing unit 325 of the mobile terminal device TA erases the image data of the still image 1810 saved in the image memory 334.

In the example of Fig. 16, input operation is performed on the shutter button 1802 in step S1030 and photographing of the still image is finished. However, the present invention is not limited to this. In step S1030, input operation may be performed twice on the video button 1803. In this case, photographing of a moving image is started by the first input operation of the video button 1803, and photographing of the moving image is finished by the second input operation. The photographing processing unit 327 temporarily saves the image data of the photographed moving image in the image memory 334 of the TA storage unit 33. In the case where a moving image is photographed, the photographed moving image is saved in the management server device SV or discarded, similarly to the case where the above-described still image is photographed.

### (Second example of care related image recording operation)

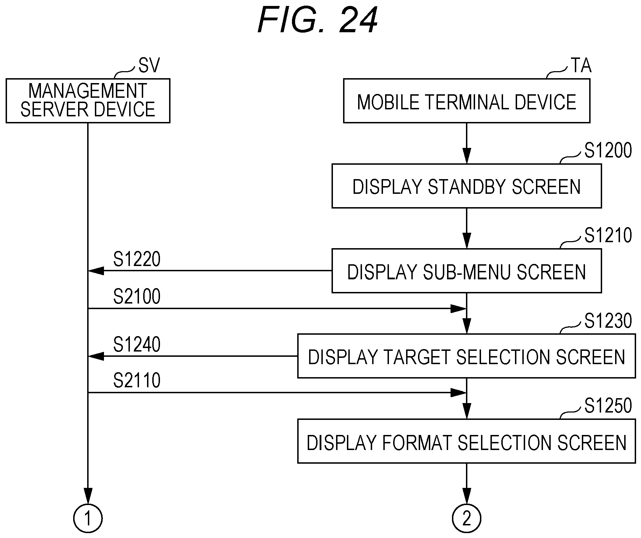

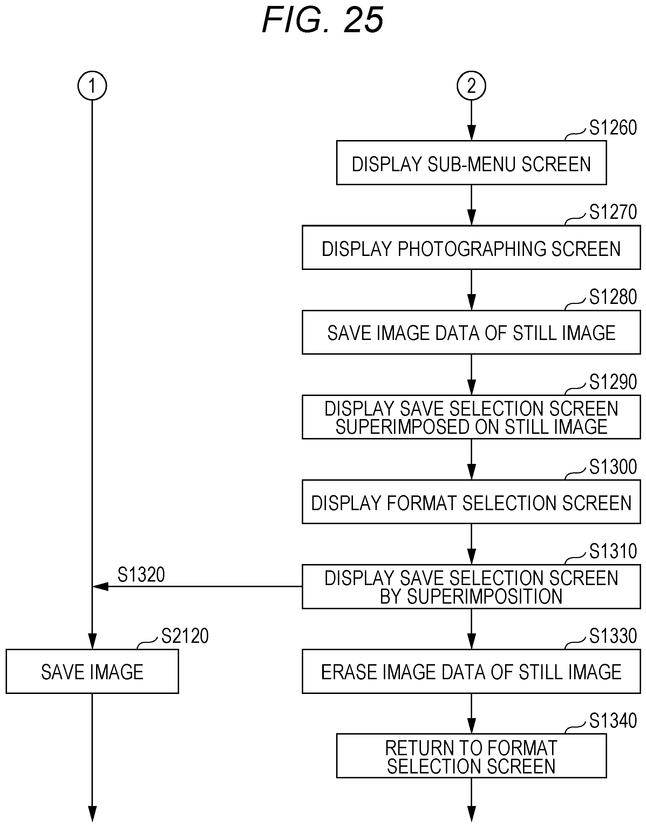

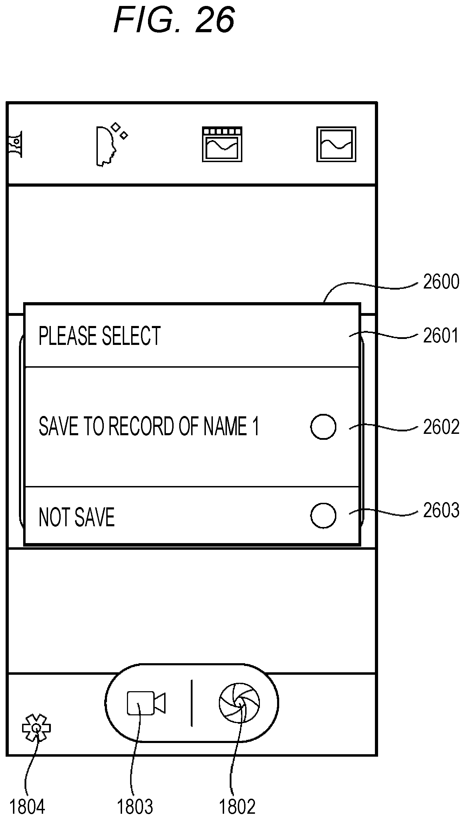

Figs. 24 and 25 are sequence diagrams schematically illustrating a second example of the care related image recording operation. Fig. 26 is a view illustrating an example of a save selection screen 2600 displayed on the mobile terminal device TA.

Step S1200 of Fig. 24 is similar to step S1000 of Fig. 16. As described above, when the login authentication is normally completed, the standby screen 51 is displayed on the TA display unit 36 of the mobile terminal device TA. When input operation (tapping) by the TA input unit 35 is performed on the sub-menu button 5111 of the menu bar area 511 in the standby screen 51 in step S1210, the TA care record processing unit 325 of the mobile terminal device TA displays the sub-menu screen 61 illustrated in Fig. 17 for example, on the TA display unit 36.

When receiving input operation of the "care implementation input" button 613 by the TA input unit 35 in a state where the sub-menu screen 61 illustrated in Fig. 17 is displayed on the TA display unit 36, the mobile terminal device TA shifts to a care record input mode. In step S1220, the TA care record processing unit 325 notifies the management server device SV of the shift to the care record input mode by the TA communication IF unit 31.

In response to this notification, in step S2100, the SV care record processing unit 224 of the management server device SV creates the target person selection screen 62, and transmits the created target person selection screen 62 to the mobile terminal device TA, similarly to the step S2000 above. In step S1230 (corresponding to an example of a monitored person selection screen display step), the TA care record processing unit 325 of the mobile terminal device TA displays the target person selection screen 62 (Fig. 20) transmitted from the management server device SV on the TA display unit 36.

When the input operation is performed on the area (the target person selection button) 622 that displays a set of the installation location and the monitored person name by the TA input unit 35 in step S1240 (corresponding to an example of a selected monitored person transmission step) in the state where the target person selection screen 62 (corresponding to an example of monitored person selection screen) is displayed and the selection of the target person has been received, the TA care record processing unit 325 stores the selected target person (corresponding to an example of a selected monitored person) in the TA storage unit 33 as a care record target person, and together with this, controls the TA communication IF unit 31 to notify the management server device SV of the selected target person, similarly to the above step S1070.

In response to this notification, in step S2110, the format distribution processing unit 223 of the management server device SV transmits the format selection screen 63 (Fig. 21) to the mobile terminal device TA. In step S1250 (corresponding to an example of the care type selection screen display step), the TA care record processing unit 325 of the mobile terminal device TA displays the format selection screen 63 (Fig. 21) transmitted from the management server device SV, on the TA display unit 36.

When input operation (tapping) by the TA input unit 35 is performed on the sub-menu button 5111 of the menu bar area 511 in step S1260 in the state where the format selection screen 63 (corresponding to an example of the care type selection screen) is displayed on the TA display unit 36, the TA care record processing unit 325 of the mobile terminal device TA displays the sub-menu screen 61 illustrated in Fig. 17 for example, on the TA display unit 36. In the subsequent steps S1270 and S1280 same as steps S1020 and S1030 in Fig. 16, the mode is shifted to the photographing mode, an image is photographed, and then, the photographed image is saved in the image memory 334.

In step S1290, the photographing processing unit 327 displays the still image 1810 saved in the image memory 334 on the TA display unit 36, and together with this, displays the save selection screen 2600 to be superimposed on the still image 1810, as illustrated in Fig. 26, for example. The save selection screen 2600 includes a message display area 2601 describing "Please select", a "Save to record of NAME 1" button 2602, and a "Not save" button 2603. When input operation is performed on the "Not save" button 2603, the photographed still image is discarded, and the display returns to the display of the format selection screen 63 (Fig. 21).

When input operation is performed on the "Save to record of NAME 1" button 2602, the TA care record processing unit 325 displays, in step S1300 (corresponding to an example of the care type selection screen redisplay step), the format selection screen 63 (corresponding to an example of the care type selection screen) illustrated in Fig. 22, obtained by adding the thumbnail image 1810T to the format selection screen 63 (Fig. 21) displayed on the TA display unit 36 in step S1250, on the TA display unit 36.

In step S1310, for example, when input operation (tapping) is performed on the "meal" area 632-1 by the TA input unit 35, the TA care record processing unit 325 displays a save selection screen 2300 to be superimposed on the format selection screen 63, as illustrated in Fig. 23. When input operation is performed on the "Not save" button 2303, the photographed image is discarded, and the display returns to the display of the format selection screen 63 illustrated in Fig. 21.

When input operation is performed on the "Save to meal record" button 2302 in step S1320 (corresponding to an example of an image transmission step, corresponding to an example of a selected care type transmission step), the TA care record processing unit 325 controls the TA communication IF unit 31 to transmit image data of the still image 1810 saved in the image memory 334 and information indicating presence of input operation (tapping) on the "Meal" area 632-1, to the management server device SV. In step S2120 (corresponding to an example of a save-to-device step), the SV care record processing unit 224 of the management server device SV saves the transmitted image data of the still image 1810 into the care record information storage unit 235 in association with the meal record (corresponding to an example of a selected care type) the monitored person's name "NAME 1".

In step S1330 (corresponding to an example of the erasing step), the TA care record processing unit 325 of the mobile terminal device TA erases the image data of the still image 1810 saved in the image memory 334. In step S1340, the display returns to the display of the format selection screen 63 illustrated in Fig. 21.

### (Third example of care related image recording operation)

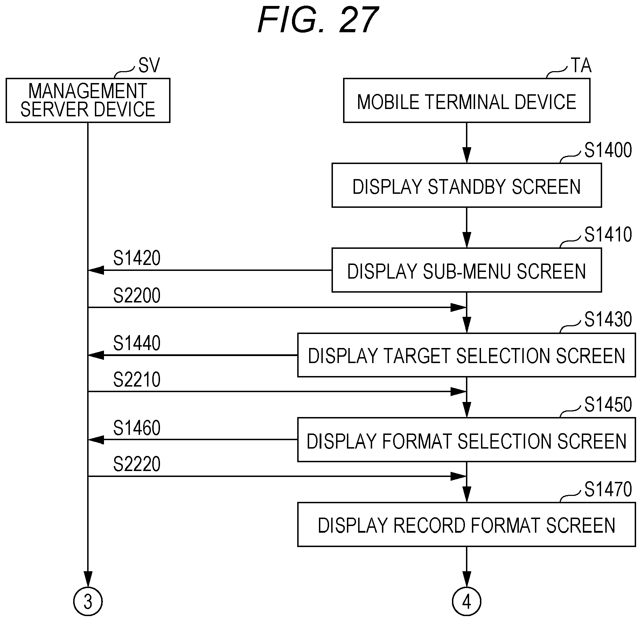

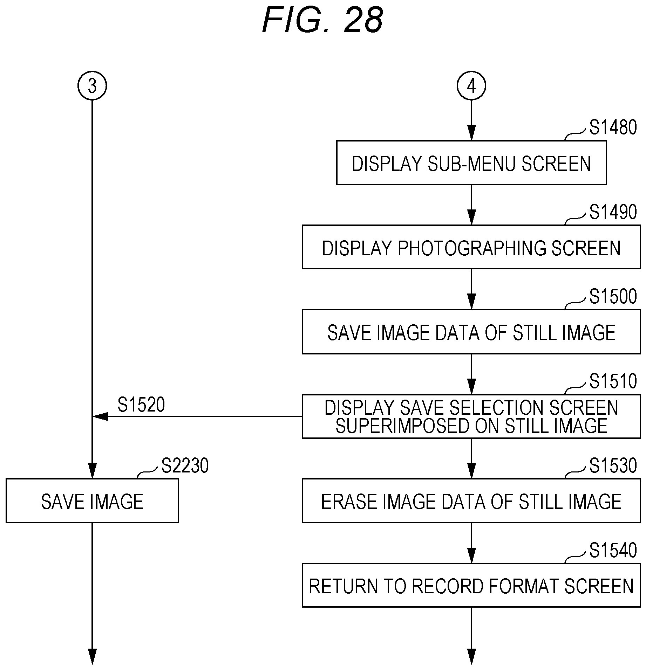

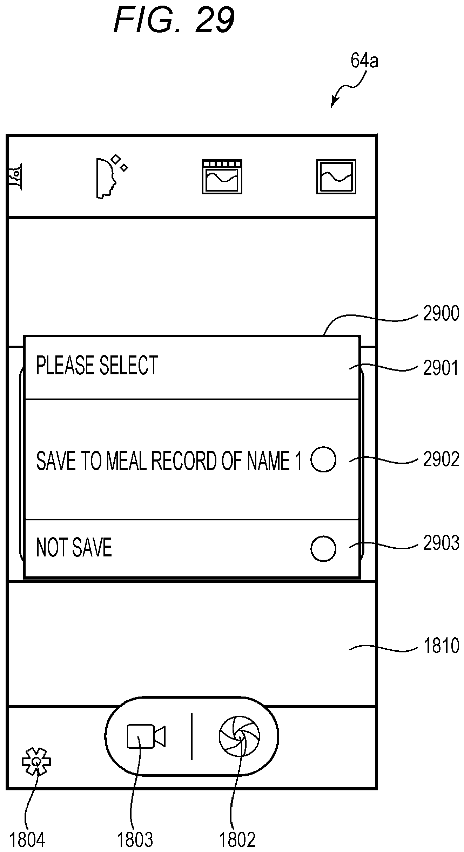

Figs. 27 and 28 are sequence diagrams schematically illustrating a third example of the care related image recording operation. Fig. 29 is a view illustrating an example of a save selection screen 2900 displayed on the mobile terminal device TA.

Step S1400 of Fig. 27 is similar to step S 1000 of Fig. 16. As described above, when the login authentication is normally completed, the standby screen 51 is displayed on the TA display unit 36 of the mobile terminal device TA. Step S1410 in Fig. 27 is similar to step S1210 in Fig. 24 in the second example. When input operation (tapping) by the TA input unit 35 is performed on the sub-menu button 5111 of the menu bar area 511 in the standby screen 51, the TA care record processing unit 325 of the mobile terminal device TA displays the sub-menu screen 61 illustrated in the above Fig. 17 for example, on the TA display unit 36.

Steps S1420, S2200, S1430, S1440, S2210, and S1450 of Fig. 27 are the same as steps S1220, S2100, S1230, S1240, S2110, and S1250 of Fig. 24 of the second example, respectively. That is, the target person selection screen 62 (Fig. 20, corresponding to an example of the monitored person selection screen in Fig. 20) is displayed on the TA display unit 36 in step S1430 (corresponding to an example of the monitored person selection screen display step); the selected target person (corresponding to an example of the selected monitored person) is notified to the management server device SV in step S1440 (corresponding to an example of the selected monitored person transmission step); and the format selection screen 63 (corresponding to an example of a care type selection screen) is displayed on the TA display unit 36 in step S1450 (corresponding to an example of a care type selection screen display step).

When input operation (tapping) is performed on the format display area 632 (for example, the "Meal" area 632-1 in the present embodiment) by the TA input unit 35 in step S1460 (corresponding to an example of a selected care type transmission step) in a state where the format selection screen 63 (Fig. 21) is displayed on the TA display unit 36, the TA care record processing unit 325 controls the TA communication IF unit 31 to notify the management server device SV of a request for a meal care recording format (corresponding to an example of a selected care type).

In response to this notification, the format distribution processing unit 223 of the management server device SV transmits in step S2220 a meal care recording format screen 64a (Fig. 6) to the mobile terminal device TA. In step S1470 (corresponding to an example of the recording format screen display step), the TA care record processing unit 325 of the mobile terminal device TA displays the meal care recording format screen 64a (Fig. 6) transmitted from the management server device SV on the TA display unit 36.

When input operation (tapping) by the TA input unit 35 is performed on the sub-menu button 5111 of the menu bar area 511 in step S1480 of Fig. 28, in a state where the meal care recording format screen 64a (Fig. 6) is displayed on the TA display unit 36, the TA care record processing unit 325 of the mobile terminal device TA displays the sub-menu screen 61 illustrated in Fig. 17 for example, on the TA display unit 36. In the subsequent steps S1490 and S1500 same as steps S1020 and S1030 in Fig. 16, the mode is shifted to the photographing mode, an image is photographed, and then, the photographed image is saved in the image memory 334.

In step S1510 (an example of the save selection screen display step), the photographing processing unit 327 displays the still image 1810 saved in the image memory 334 on the TA display unit 36, and together with this, displays the save selection screen 2900 over the still image 1810, as illustrated in Fig. 29, for example. The save selection screen 2900 includes a message display area 2901 describing "Please select", a "Save to meal record of NAME 1" button 2902, and a "Not save" button 2903. When input operation is performed on the "Not save" button 2903, the photographed still image is discarded and the display returns to the display of the meal care recording format screen 64a (Fig. 6).

When input operation is performed on the "Save to meal record of NAME 1" button 2902, the TA care record processing unit 325 controls the TA communication IF unit 31 to transmit image data of the still image 1810 saved in the image memory 334 to the management server device SV in step S1520 (an example of the image transmission step). In step S2230 (an example of a save-to-device step), the SV care record processing unit 224 of the management server device SV saves the transmitted image data of the still image 1810 into the care record information storage unit 235 in association with the meal record of the monitored person's name "NAME 1".

In step S1530 (an example of the erasing step), the TA care record processing unit 325 of the mobile terminal device TA erases the image data of the still image 1810 saved in the image memory 334. In step S1540, the display returns to the display of the meal care recording format screen 64a (Fig. 6).

### (Fourth example of care related image recording operation)

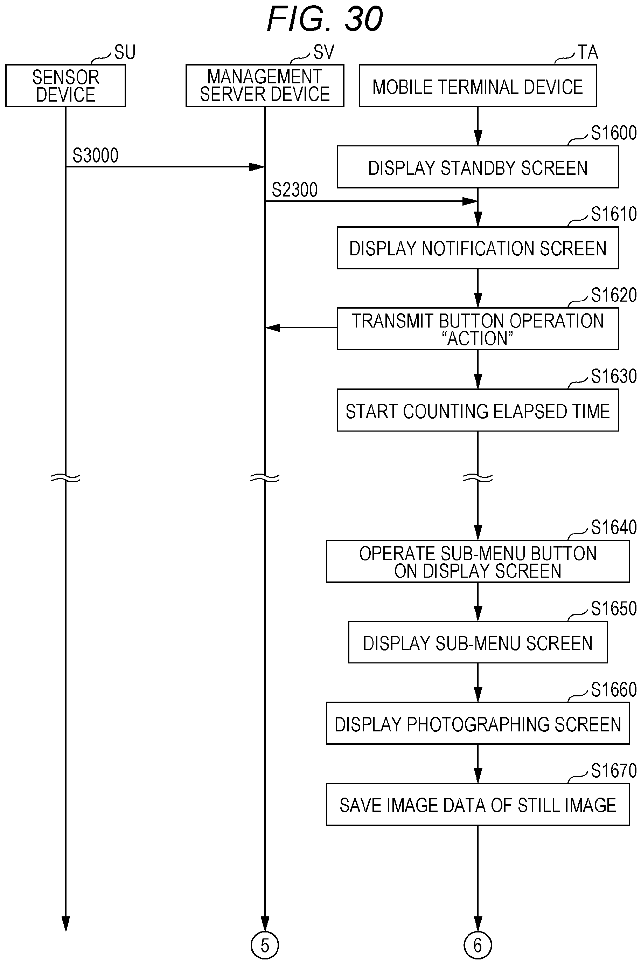

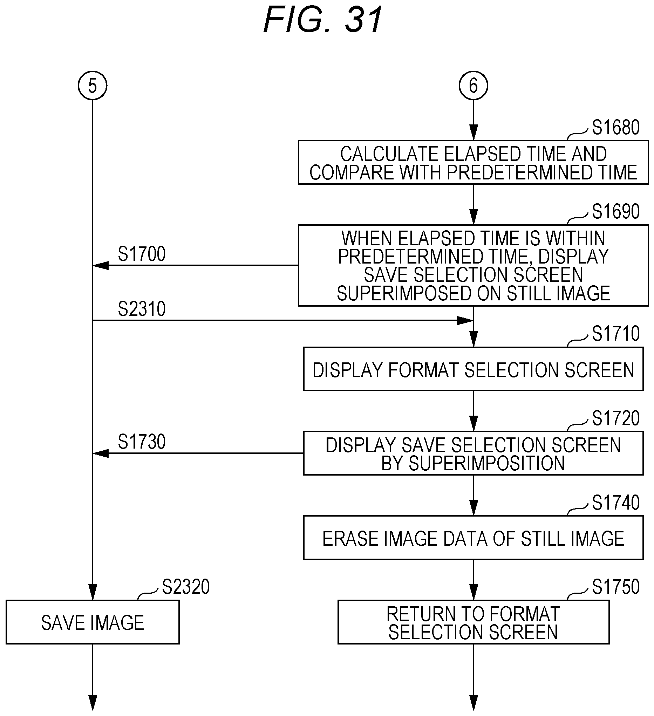

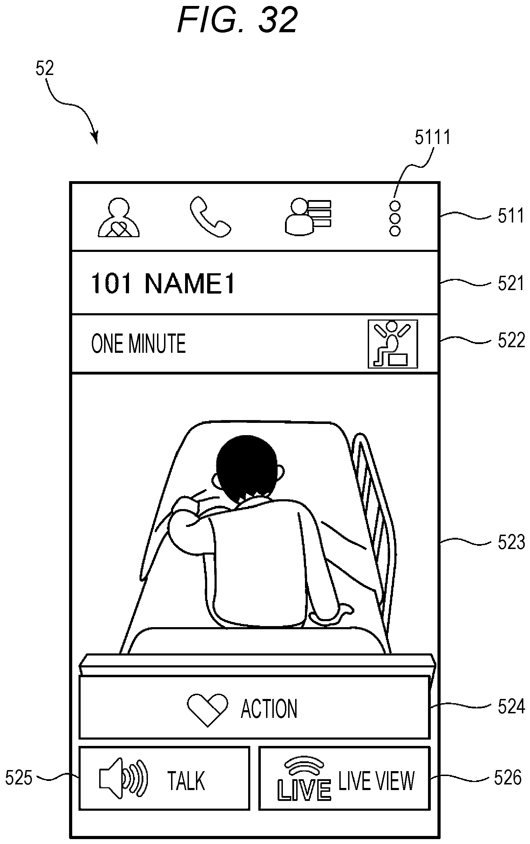

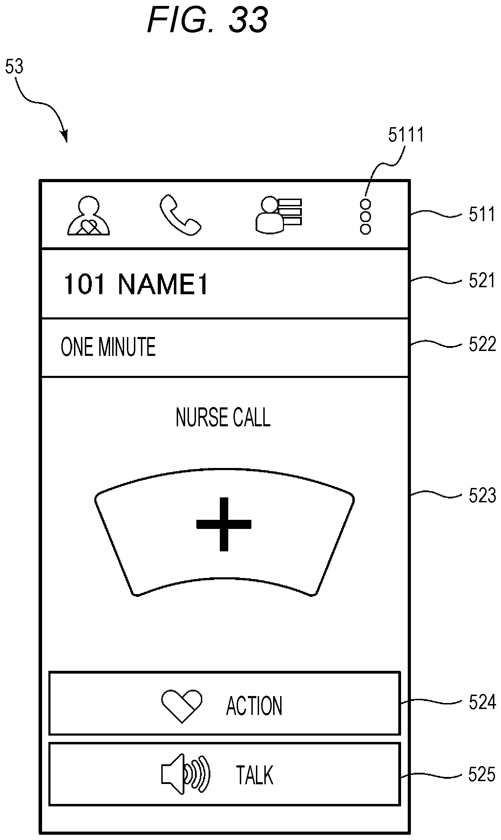

Figs. 30 and 31 are sequence diagrams schematically illustrating a fourth example of the care related image recording operation. Fig. 32 is a view illustrating an example of the monitoring information screen 52 displayed on the mobile terminal device TA. Fig. 33 is a view illustrating an example of the nurse call reception screen 53 displayed on the mobile terminal device TA.

The sensor device SU operates as follows at regular time intervals (for example, one second) and thereby detects predetermined operation in the monitored person Ob, and determines the presence or absence of reception of a nurse call. First, the sensor device SU obtains a one-frame image (image data) as a target image from the image sensor, and detects a predetermined behavior of the monitored person Ob on the basis of the obtained target image. In response to detection of the predetermined behavior, the sensor device SU transmits a first event notification communication signal regarding the detection of the predetermined behavior and containing the predetermined behavior detected as the event information to the management server device SV in step S3000 of Fig. 30.

While operating in this manner, the sensor device SU determines whether a nurse call has been received. When the nurse call is received, the sensor device SU transmits, in step S3000 of Fig. 30, the first event notification communication signal regarding the reception of the nurse call, which contains the nurse call received as the event information, to the management server device SV.

When receiving the first event notification communication signal from the sensor device SU via the network NW, the SV monitoring processing unit 222 of the management server device SV stores various types of information such as the sensor ID and the event information contained in the first event notification communication signal in the SV monitoring information storage unit 231 as monitoring information of the monitored person Ob monitored by the sensor device SU having this sensor ID.

Subsequently, the SV monitoring processing unit 222 of the management server device SV determines the terminal device SP or TA being the notification destination corresponding to the sensor device SU as the notification source in the received first event notification communication signal on the basis of the notification destination correspondence relationship. In the example of Fig. 30, the mobile terminal device TA is determined. In step S2300, the management server device SV transmits a second event notification communication signal to the identified mobile terminal device TA as the notification destination.

When receiving the second event notification communication signal from the management server device SV via the network NW, the TA monitoring processing unit 322 of the mobile terminal device TA stores various types of information such as the sensor ID and the event information contained in the second event notification communication signal in the TA monitoring information storage unit 331 as monitoring information of the monitored person Ob monitored by the sensor device SU having this sensor ID, and displays the monitoring information on the TA display unit 36.

Meanwhile, step S1600 of Fig. 30 is similar to step S1000 of Fig. 16. As described above, when the login authentication is normally completed, the standby screen 51 is displayed on the TA display unit 36 of the mobile terminal device TA. While the standby screen 51 that waits for a communication signal addressed to the own device is displayed on the TA display unit 36, the TA control unit 321 of the TA control processing unit 32 of the mobile terminal device TA repeats determination whether a communication signal has been received by the TA communication IF unit 31 in step S1600 (corresponding to an example of a monitoring information reception step) until the communication signal is received. When the communication signal is received, the mobile terminal device TA determines the type of the received communication signal.

In a case where the received communication signal is not the second event notification communication signal as a result of the determination, the mobile terminal device TA performs appropriate processing according to the received communication signal to complete the present processing. In contrast, in a case where the received communication signal is the second event notification communication signal, the TA monitoring processing unit 322 of the TA control processing unit 32 of the mobile terminal device TA performs, in step S1610 (corresponding to an example of an action selection screen display step) operation of storing monitoring information regarding monitoring of the monitored person Ob, contained in the received second event notification communication signal, into the TA monitoring information storage unit 331, and then displaying a notification screen according to individual information contained in the received second event notification communication signal, on the TA display unit 36.

More specifically, in step S1610, in a case where the event information contained in the received second event notification communication signal is the predetermined behavior, the TA monitoring processing unit 322 displays, for example, the monitoring information screen 52 illustrated in Fig. 32 (corresponding to an example of action selection screen) on the TA display unit 36 as a notification screen. In contrast, in a case where the event information contained in the received second event notification communication signal is the nurse call, the TA monitoring processing unit 322 displays, for example, a nurse call reception screen 53 (corresponding to an example of an action selection screen) illustrated in Fig. 33 on the TA display unit 36 as a notification screen.

The monitoring information screen 52 is a screen for displaying the monitoring information related to the monitoring of the monitored person Ob. For example, as illustrated in Fig. 32, the monitoring information screen 52 includes: a menu bar area 511; a monitored person name area 521 that displays the installation location of the sensor device SU having the sensor ID contained in the received second event notification communication signal and the name of the monitored person Ob monitored by the sensor device SU having the sensor ID; a detection information display area 522 that displays elapsed time from the reception time of the received second event notification communication signal or the detection time of the predetermined behavior and that displays the event information contained in the received second event notification communication signal, that is, detection results of the predetermined behavior; an image area 523 that displays an image contained in the received second event notification communication signal, that is, a target image being a still image, for example, photographed by the sensor device SU having the sensor ID; an "Action" button 524; a "Talk" button 525; and a "LIVE view" button 526.

In order to display the installation location of the sensor device SU and the name of the monitored person Ob in the monitored person name area 521, the installation location of the sensor device SU and the name of the monitored person Ob are retrieved from the TA sensor information storage unit 332 and displayed, by using the sensor ID contained in the received second event notification communication signal, as a retrieval key.

The detection information display area 522 may display the detection result (names of sitting-up, bed-leaving, falling and tumbling in the present embodiment) as they are, contained in the received second event notification communication signal. In the present embodiment, however, icons that symbolically represent the detection results are used. In order to perform display using the icons, the TA storage unit 33 preliminarily stores individual behaviors associated with icons that symbolically represent the behaviors. In an example illustrated in Fig. 32, the detection information display area 522 displays a sitting-up icon that symbolizes the sitting-up.

In the monitoring information screen 52, the "Action" button 524 is a button for inputting, to the mobile terminal device TA, that the user of the mobile terminal device TA has an intention to implement a predetermined action such as life-saving, nursing, caregiving and assistance in response to the detection result displayed on the monitoring information screen 52, for example.

The "Talk" button 525 is a button for requesting voice calling, and is used to input an instruction to communicably connect the sensor device SU of the sensor ID with the mobile terminal device TA via the network NW. The "LIVE view" button 526 is a button for requesting a live moving image, and is a button for inputting an instruction to display a moving image captured by the sensor device SU of the sensor ID.

The nurse call reception screen 53 is a screen for displaying reception of a nurse call. For example, as illustrated in Fig. 33, the nurse call reception screen 53 displays a menu bar area 511, a monitored person name area 521, a detection information display area 522, a nurse call reception notification display area 531 that displays a message ("Nurse call)", for example in Fig. 33) indicating that a nurse call has been received, an "Action" button 524, and a "Talk" button 525. The nurse call reception screen 53 simply displays the elapsed time from the previous reception time at which the received second event notification communication signal or from the reception time of the nurse call, in the detection information display area 522. The nurse call reception screen 53 may further include the "LIVE view" button 526 (Fig. 32).

Subsequently, during the display of the monitoring information screen 52 and the nurse call reception screen 53 in this manner, the TA control processing unit 32 of the mobile terminal device TA repeatedly determines whether input operation has been received at a touch panel with the TA input unit 35 and the TA display unit 36 until any input operation is received. When input operation is received, the TA control processing unit 32 of the mobile terminal device TA executes appropriate processing according to the content of the input operation to complete the present processing.

For example, when receiving input operation on the "Action" button 524 from the TA input unit 35 included in the touch panel, the TA control processing unit 32 of the mobile terminal device TA first adds information of reception of "Action" to the monitoring information of the monitored person Ob currently displayed on the TA display unit 36, and then stores the information in the TA monitoring information storage unit 331. More specifically, the TA control processing unit 32 registers an action flag "1" representing reception of intention of action to an action field 3316 of a record (here, the record that has registered the monitoring information contained in the received second event notification communication signal) that has registered the monitoring information of the monitored person Ob currently displayed on the TA display unit 36, on the TA monitoring information table MT-TA stored in the TA monitoring information storage unit 331.

Subsequently, in step S1620 of Fig. 30, the TA control processing unit 32 transmits an action reception notification communication signal containing the sensor ID corresponding to the monitoring information of the monitored person Ob displayed on the TA display unit 36 and action reception information indicating reception of "Action", to the management server device SV. When receiving this action reception notification communication signal, the SV control processing unit 22 of the management server device SV transmits an action reception comprehension communication signal containing the sensor ID and the action reception information contained in the received action reception notification communication signal, to the terminal devices SP and TA by multicasting communication. This allows synchronization of the information of reception of "Action" among the terminal devices SP and TA, regarding the sensor ID corresponding to the monitoring information of the monitored person Ob displayed on the TA display unit 36.

Furthermore, for example, when receiving input operation of the "Talk" button 525, the TA control processing unit 32 of the mobile terminal device TA notifies this information to the call processing unit 323. The call processing unit 323 transmits a call request communication signal containing information such as a request for voice calling to the sensor device SU that monitors the monitored person Ob displayed on the TA display unit 36, so as to achieve voice-call ready connection with the sensor device SU that has responded to this signal, via the network NW. This enables voice calling between the mobile terminal device TA and the sensor device SU.

When receiving input operation of the "Finish" button (not illustrated) which is a button for inputting an instruction to finish the voice calling, the TA control processing unit 32 of the mobile terminal device TA notifies the information to the call processing unit 323. The call processing unit 323 transmits a call finish communication signal containing information such as a request to finish the voice calling to the sensor device SU that monitors the monitored person Ob displayed on the TA display unit 36. This finishes the voice calling between the mobile terminal device TA and the sensor device SU.

Furthermore, for example, when receiving input operation of the "LIVE view" button 526, the TA control processing unit 32 of the mobile terminal device TA notifies this information to the TA streaming processing unit 324. The TA streaming processing unit 324 transmits a moving image distribution request communication signal containing information such as a request for distribution of live moving image to the sensor device SU that monitors the monitored person Ob currently displayed on the TA display unit 36 so as to achieve moving-image downloadable connection of the TA streaming processing unit 324 via the network NW with the sensor device SU that has responded to this signal. When receiving distribution of a live moving image from the sensor device SU, the TA streaming processing unit 324 displays the moving image included this distribution in streaming reproduction on the TA display unit 36.

The monitoring information screen 52 that displays the live moving image displays the moving image in the image area 523 and displays a "Finish LIVE view" button (not illustrated) instead of the "LIVE view" button 526. With this configuration, a live moving image is displayed on the mobile terminal device TA. The "Finish LIVE view" button (not illustrated) is a button for requesting finishing a live moving image, and is a button for inputting an instruction to finish distribution of a moving image captured by the sensor device SU of the sensor ID and to finish display of the moving image.

When receiving input operation of the "Finish LIVE view" button, the TA control processing unit 32 of the mobile terminal device TA notifies this information to the TA streaming processing unit 324. The TA streaming processing unit 324 transmits a moving image distribution finish communication signal containing information such as a request for finishing the moving image distribution to the sensor device SU that monitors the monitored person Ob currently displayed on the TA display unit 36, and displays the still image on the TA display unit 36. With this operation, the mobile terminal device TA finishes the live display of moving image.

With the operation, the assistance system MS detects a predetermined behavior in each of the monitored persons Ob generally by each of the sensor devices SU, the management server device SV, the fixed terminal device SP and the mobile terminal device TA, and receives nurse call to monitor each of the monitored persons Ob.

Furthermore, in step S1630 (corresponding to an example of a time counting step) of Fig. 30 in the present embodiment, the TA control processing unit 32 of the mobile terminal device TA uses a timer function of the CPU to start to count the elapsed time from the time of reception of the input operation on the "Action" button 524. Thereafter, in response to input operation (tapping) on the sub-menu button 5111 on the display screen of the TA display unit 36 (step S1640), the TA care record processing unit 325 displays, for example, the sub-menu screen 61 illustrated in Fig. 17 on the TA display unit 36 (step S1650). The display screen of the TA display unit 36 on which input operation (tapping) on the sub-menu button 5111 is performed in step S1640 is, for example, the format selection screen 63 (Fig. 21), the recording format screen 64 (for example, meal care recording format screen 64a in Fig. 6). In the subsequent steps S1660 and S1670 same as steps S1020 and S1030 in Fig. 16, the mode is shifted to the photographing mode, an image is photographed, and then, the photographed image is saved in the image memory 334.

In step S1680 of Fig. 31, the TA control processing unit 32 calculates the elapsed time from the time of receiving the input operation of the "Action" button 524, and compares the time with a predetermined time. In a case where the elapsed time is within the predetermined time in step S1690 (corresponding to an example of the save selection screen display step), the photographing processing unit 327 displays the still image 1810 saved in the image memory 334 on the TA display unit 36, and together with this, displays the save selection screen 2600 over the still image 1810, as illustrated in above Fig. 26, for example. In the example of Figs. 32 and 33, since the target person is "NAME 1", the "Save to record of NAME 1" button 2602 is displayed as illustrated in Fig. 26. When input operation is performed on the "Not save" button 2603, the photographed still image is discarded, and the display returns to the display screen displayed on the TA display unit 36 at execution of input operation (tapping) on the sub-menu button 5111 in step S1640.

When input operation is performed on the "Save to record of NAME 1" button 2602, the TA care record processing unit 325 controls the TA communication IF unit 31 to notify the management server device SV of a transmission request for the format selection screen 63 (Fig. 21) in step S1700.

In response to this notification, the format distribution processing unit 223 of the management server device SV transmits the format selection screen 63 (Fig. 21) to the mobile terminal device TA in step S2310. In step S1710, the TA care record processing unit 325 of the mobile terminal device TA displays the format selection screen 63 (Fig. 21) transmitted from the management server device SV on the TA display unit 36.

The following steps S1720, S1730, S1740, S1750, and S2320 are the same as steps S1310, S1320, S1330, S1340, and S2120 of Fig. 25 of the second example, respectively.

That is, in step 1730 (corresponding to an example of an image transmission step), image data of the still image 1810 saved in the image memory 334 and the information representing the area that has received input operation (tapping) on the format selection screen 63 are transmitted to the management server device SV. In step S1740 (corresponding to an example of an erasing step), the image data of the still image 1810 saved in the image memory 334 is erased. In step S2320 (corresponding to an example of a save-to-device step), the transmitted image data of the still image 1810 is saved in the care record information storage unit 235 in association with, for example, the monitored person name "NAME 1" and the care type corresponding to the area on the format selection screen 63 where the input operation (tapping) is performed.

Examples of the predetermined time to be compared with the elapsed time in step S1680 include three minutes, five minutes, 15 minutes, etc., and any time can be predetermined. The predetermined time may be varied for each of types of notification from the management server device SV to the mobile terminal device TA. For example, in the case of notification of a nurse call (Fig. 33), the predetermined time may be set to three minutes. For example, in the case of sitting-up, bed-leaving, etc. among notifications of monitoring information (Fig. 32), the predetermined time may be set to five minutes. For example, in the case of falling from bedding, tumbling during walking, or the like in a notification of monitoring information (Fig. 32), the predetermined time may be set to 15 minutes.

In addition, in a case where the image is photographed and saved within the predetermined time, the image may be saved in association with the notification record saved in the management server device SV or the image photographed by the sensor device SU and saved together with the detection.

### (Viewer screen displayed on fixed terminal device)

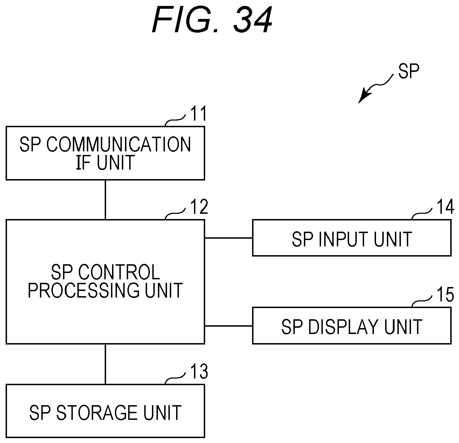

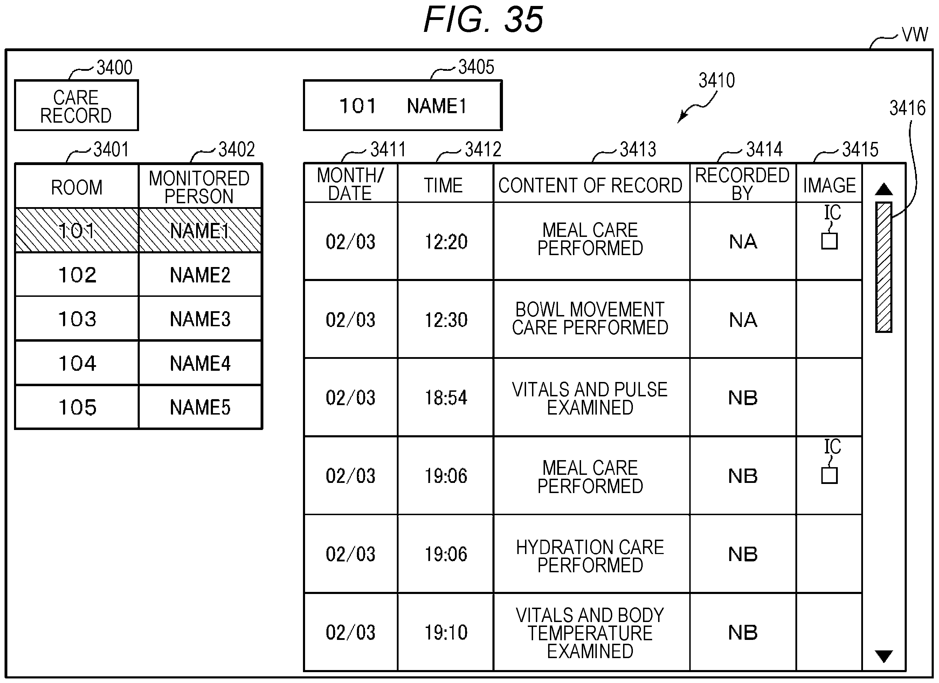

Fig. 34 is a block diagram illustrating a configuration of the fixed terminal device SP. Fig. 35 is a diagram illustrating an example of a viewer screen VW of a care record displayed on the fixed terminal device SP. The viewer screen displayed on the fixed terminal device will be described with reference to Figs. 34 and 35.

In the present embodiment, as illustrated in Fig. 34, the fixed terminal device SP includes an SP communication IF unit 11, an SP control processing unit 12, an SP storage unit 13, an SP input unit 14, and an SP display unit 15, for example.

The SP input unit 14 is connected to the SP control processing unit 12 and includes a mouse and a keyboard, for example. The SP display unit 15 is a circuit that is connected to the SP control processing unit 12 and displays the operation content input from the SP input unit 14 and the viewer screen VW or the like under the control of the SP control processing unit 12. Examples of the SP display unit 15 include display devices such as an LCD or an organic EL display.

The SP communication IF unit 11 is a communication circuit that is connected to the SP control processing unit 12 and performs communication under the control of the SP control processing unit 12. The SP communication IF unit 11 includes, for example, a communication interface circuit conforming to the same standard for the SV communication IF unit 21 and the TA communication IF unit 31, such as the IEEE 802.11 standard.

The SP storage unit 13 is a circuit that is connected to the SP control processing unit 12 and stores various predetermined programs and various predetermined data. Examples of the various predetermined programs include control processing programs such as an SP control program that controls individual portions of the fixed terminal device SP according to the function of the individual portions, and a screen creation processing program that creates the viewer screen VW. The SP storage unit 13 includes a ROM and an EEPROM, for example. The SP storage unit 13 includes, for example, a RAM serving as a working memory of the SP control processing unit 12 for storing data or the like generated during execution of the predetermined program.

The SP control processing unit 12 is configured to include, for example, a CPU and its peripheral circuits. The SP control processing unit 12 executes the control processing program and thereby creates the viewer screen VW using the information stored in the management server device SV, and then, displays the created viewer screen VW on the SP display unit 15. The staff of the nurse station ST (Fig. 1) can confirm information saved in the management server device SV by viewing the viewer screen VW displayed on the fixed terminal device SP.

As illustrated in Fig. 34, a title display area 3400 is provided at the upper left of the viewer screen VW. The title display area 3400 displays a title of the viewer screen VW. In the example of Fig. 34, "Care record" is displayed in the title display area 3400, indicating that the viewer screen VW of Fig. 34 is a screen displaying the care record.

Immediately below the title display area 3400, a room display area 3401 and a monitored person name display area 3402 are provided. The room display area 3401 displays a room number indicating the room accommodating the monitored person Ob. The monitored person name display area 3402 displays the name of the monitored person using the room with the room number displayed in the corresponding room display area 3401.

A display target person display area 3405 is provided at the upper center of the viewer screen VW, and a care record display area 3410 is provided below the display target person display area 3405. The display target person display area 3405 displays the name of the monitored person who is the display target of the care record displayed in the care record display area 3410.

The care record display area 3410 includes a date display area 3411, a time display area 3412, a record content display area 3413, a recording person display area 3414, an image display area 3415, and a scroll bar 3416. The date display area 3411 and the time display area 3412 respectively display the date and time when care is given to the monitored person Ob. The record content display area 3413 displays the content of the care record. The recording person display area 3414 displays a recording person who created the care record. The image display area 3415 displays whether the care related image photographed by the camera 38 of the mobile terminal device TA is included as a care record. The scroll bar 3416 can be used to scroll the display content of the care record display area 3410.

An icon IC displayed in the image display area 3415 indicates that a care related image photographed by the camera 38 of the mobile terminal device TA is included as a care record. The icon IC includes a link to the care related image stored in the management server device SV. That is, the SP control processing unit 12 of the fixed terminal device SP is configured to obtain a care related image from the management server device SV and display the obtained care related image on the SP display unit 15 in response to operation on the icon IC using the SP input unit 14 by the staff of the nurse station ST (Fig. 1). A thumbnail image of the care related image may be displayed as the icon IC in the image display area 3415.

### (Effects)

As described above, in the first example of the care related image recording operation, the camera 38 is activated and the care related image is photographed from a state where the standby screen 51 is displayed on the TA display unit 36. Therefore, in a case where the care related image is saved, the target person being the monitored person Ob to be the target for saving of care related images in association and the target care being the type of care to be the target for saving of the care related images in association are not determined and thus unknown.

Accordingly, in the first example of the care related image recording operation, after the care related image is photographed, the target person selection screen 62 is displayed on the TA display unit 36. When the target person is selected following the target person selection screen 62, the format selection screen 63 will be displayed on the TA display unit 36. Subsequently, in addition to the care related image, the target person selected following the target person selection screen 62 and the type of care selected following the format selection screen 63 are transmitted from the mobile terminal device TA to the management server device SV.

The care related image is stored in the management server device SV as a care record in association with the selected target person and the type of care. Therefore, according to the first example of the care related image recording operation, it is possible to save the care related image as a care record in association with both the target person and the type of care in the management server device SV even in a case where the camera 38 is activated and the care related image is photographed from the state where the standby screen 51 is displayed on the TA display unit 36.

Additionally, in the second example of the care related image recording operation, the target person selection screen 62 is displayed on the TA display unit 36. When the target person is selected following the target person selection screen 62, the format selection screen 63 will be displayed on the TA display unit 36. Subsequently, the camera 38 is activated and a care related image is photographed before the type of care is selected following the format selection screen 63 in a state where the format selection screen 63 is displayed on the TA display unit 36. In this case, the monitored person who is the target for saving of the care related image in association is highly likely to be the target person selected following the target person selection screen 62.

Accordingly, in the second example of the care related image recording operation, the care related image is saved in the management server device SV as a care record in association with the target person selected following the target person selection screen 62. Therefore, according to the second example, there is an advantage of being able to omit time and effort to reselect the monitored person Ob to be the target to be the target for saving associated with the care related image after photographing the care related image.

Furthermore, in the second example of the care related image recording operation, the format selection screen 63 is displayed on the TA display unit 36 again after the care related image is photographed, enabling selection of the type of care. Therefore, according to the second example, it is possible to save the care related image in the management server device SV as a care record in association with both the selected target person and the type of care.

In the third example of the care related image recording operation, when the target person selection screen 62 is displayed on the TA display unit 36 and the target person following the target person selection screen 62 is selected, the format selection screen 63 is displayed on the TA display unit 36. When the care type is selected following the format selection screen 63, the recording format screen 64 is displayed on the TA display unit 36. Subsequently, the camera 38 is activated and a care related image is photographed in a state where the recording format screen 64 is displayed on the TA display unit 36, In this case, the monitored person who is the target for saving of the care related image in association is a target person selected following the target person selection screen 62 and the type of care for which care related images are to be saved in association is highly likely to be the type of care selected following the format selection screen 63.

Therefore, in the third example of the care related image recording operation, the care related image is saved in the management server device SV as a care record in association with the target person selected following the target person selection screen 62 and the type of care selected following the format selection screen 63. Therefore, according to the third example, there is an advantage of being able to omit time and effort to reselect the monitored person Ob to be the target to be associated and saved with the care related image and the type of care with which the care related image is to be saved in association, after photographing the care related image. Moreover, it is possible to save the care related image in the management server device SV as a care record in association with both the selected target person and the type of care.

In the fourth example of the care related image recording operation, the camera 38 is activated and a care related image is photographed within a predetermined time from the time of reception of the input operation on the "Action" button 524 on either the monitoring information screen 52 or the nurse call reception screen 53 displayed on the TA display unit 36 as the notification screen. In this case, the save selection screen 2600 displayed on the TA display unit 36 prompts selection of whether to save the care related image in association with the monitored person Ob corresponding to the monitoring information screen 52 or the nurse call reception screen 53.

When the selection of saving a care related image is made, the care related image is saved in the management server device SV as a care record in association with the monitored person Ob corresponding to the monitoring information screen 52 or the nurse call reception screen 53 and with a result of the action being a result obtained by taking action for the monitored person Ob. Therefore, according to the fourth example of the care related image recording operation, it is sufficient to simply select, after a care related image is photographed, operation of saving the care related image in the management server device SV in association with the monitored person Ob following the save selection screen 2600. This produces an advantage that it is not necessary to reselect the monitored person Ob to be the target for saving associated with the care related image.

Furthermore, in the fourth example of the care related image recording operation, the format selection screen 63 is displayed on the TA display unit 36 after the care related image is photographed, enabling selection of the type of care. Therefore, according to the fourth example, it is possible to save the care related image in the management server device SV as a care record in association with both the monitored person Ob and the type of care.

Furthermore, in any of the first to fourth examples of the care related image recording operation, it is possible to select either one of operation of saving the care related image as a care record or operation of discarding the care related image without being saved. In addition, even in the case where the image is to be saved as a care record, the image data transmitted from the mobile terminal device TA to the management server device SV and then temporarily saved in the image memory 334 of the mobile terminal device TA is to be erased in the present configuration. In this manner, the present embodiment is configured to prohibit saving of a care related image except for the occasion of saving the image as a care record in the management server device SV. This makes it possible to protect the privacy of the monitored person Ob whose image is captured.

As described above, according to the present embodiment, after photographing a care related image, it is possible to save the care related image as a care record by a simple procedure simply by selecting to save the care related image.

### (Others)

(1) In the first to fourth examples of the recording operation of the care related image, an image obtained by photographing a status of remaining food after meal at the time of meal care of the monitored person Ob is used as a specific example of the care related image. However, the care related image of the present embodiment is not limited to this. Alternatively, as the care related image, an image obtained by capturing the condition of bowl movement when bowl movement care is applied to the monitored person Ob. Further alternatively, it is also adopt an image of an affected part of injury or disease of the monitored person Ob or adopt a moving image or the like of the monitored person Ob when acting abnormally, as a care related image.
   Regarding these, as in the present embodiment, it is possible to create a care record in a short time by saving the photographed image as a care record just by simple operation. For example, it would be conceivable to provide a workflow of reducing the number of man-hours of the monitoring person by adopting creation of a care record using an image as a basic procedure and thereafter creating a care record in text while viewing the image in a case where a care record in text is needed.
(2) In the first example of the care related image recording operation, the care related image is saved in the management server device SV in association with both the target person and the type of care. However, the present invention is not limited to this example. For example, the care related image may be saved in the management server device SV in association only with the target person.

For example, when the input operation on the area 622 (that is, target person selection button) that displays a set of the installation location and the monitored person name is performed by the TA input unit 35 in a state where the target person selection screen 62 is displayed on the TA display unit 36 in step S1060 (Fig. 16) and the selection of the target person has been received, the TA care record processing unit 325 may display the save selection screen 2600 (Fig. 26) to be superimposed on the target person selection screen 62.

When input operation is performed by the TA input unit 35 on the "Save to record of NAME 1" button 2602, the TA care record processing unit 325 may control the TA communication IF unit 31 to transmit image data of the still image 1810 saved in the image memory 334 and information indicating that the input operation has been performed on the "Save to record of NAME 1" button 2602 to the management server device SV. The SV care record processing unit 224 of the management server device SV may save the transmitted image data of the still image 1810 into the care record information storage unit 235 in association with the record of the monitored person's name "NAME 1".

While the present description discloses technologies of various aspects as described above, the main technologies among them will be summarized below.

A first aspect of the present invention is
an assistance method used for an assistance system that assists a caregiver who gives care to a monitored person,
the assistance system including:
a central processing unit that saves a care record that records content of care given to the monitored person by the caregiver; and
a terminal device including an imaging unit that photographs an image, a display unit that displays the image photographed by the imaging unit, and an input unit that inputs the care record, and communicably connected to the central processing unit,
the assistance method including:
   an imaging step of activating the imaging unit and photographing a care related image being an image related to the content of the care given by the caregiver;
   a save selection screen display step of displaying, after photographing of the care related image, the photographed care related image and a save selection screen that prompts selection of whether to save the care related image in the central processing unit, on the display unit;
   an image transmission step of transmitting the care related image from the terminal device to the central processing unit after selection of saving of the care related image in the central processing unit in accordance with the save selection screen; and
   a save-to-device step of saving the care related image as the care record in the central processing unit after transmission of the care related image.

A second aspect of the present invention is
an assistance system that assists a caregiver who gives care to a monitored person,
the assistance system including:
a central processing unit that saves a care record that records content of care given to the monitored person by the caregiver; and
a terminal device including an imaging unit that photographs an image, a display unit that displays the image photographed by the imaging unit, and an input unit that inputs the care record, and communicably connected to the central processing unit,
the assistance system further including:
a photographing processing unit that activates the imaging unit, photographs a care related image being an image related to the content of the care given by the caregiver, and displays the photographed care related image and a save selection screen that prompts selection of whether to save the care related image in the central processing unit, on the display unit;
an image transmission processing unit that transmits the care related image from the terminal device to the central processing unit after selection of saving of the care related image in the central processing unit in accordance with the save selection screen; and
a save-to-device processing unit that saves the care related image as the care record in the central processing unit after transmission of the care related image.

In the first and second aspects, the imaging unit is activated and a care related image that is an image related to the content of care given by the caregiver is photographed, and the photographed care related image is displayed on the display unit. When the care related image is displayed on the display unit, the save selection screen that prompts selection of whether to save the care related image in the central processing unit is automatically displayed on the display unit. When saving of the care related image in the central processing unit is selected in accordance with the save selection screen, the care related image is automatically transmitted from the terminal device to the central processing unit and saved as a care record. Therefore, according to the first and second aspects, after photographing a care related image, it is possible to save the care related image as a care record by a simple procedure simply by selecting to save the care related image.

In the first aspect, it is allowable, for example, to further include:
a standby screen display step of displaying a standby screen on the display unit;
a target person selection screen display step of displaying, on the display unit, a target person selection screen for selecting a target person being the monitored person as a target to be saved in association with the care related image after the imaging step and the save selection screen display step are executed from a state where the standby screen is displayed on the display unit, and saving the care related image in the central processing unit is selected in accordance with the save selection screen; and
a target person transmission step of transmitting information representing a selected target person being the target person who has been selected, from the terminal device to the central processing unit after the target person is selected in accordance with the target person selection screen.

The save-to-device step may save the care related image in association with the selected target person as the care record in the central processing unit.

In a case where the imaging step and the save selection screen display step are executed from the state where the standby screen is displayed on the display unit, the monitored person to be a target for saving in association with the care related image is not determined and thus unknown.

In contrast, in the present aspect, the target person selection screen for selecting the target person is displayed on the display unit and when the target person is selected in accordance with the target person selection screen, the information representing the selected target person is automatically transmitted from the terminal device to the central processing unit. In addition, the care related image is saved in the central processing unit as a care record in association with the selected target person. Therefore, according to the present aspect, it is possible to save the care related image as a care record in the central processing unit in association with the selected target person.

In the first aspect, it is allowable, for example, to further include:
a target care type selection screen display step of displaying, on the display unit, a target care type selection screen for selecting a target care type which is a type of care to be saved in association with the care related image after transmission of information representing the selected target person from the terminal device to the central processing unit; and
a selected target care type transmission step of transmitting information representing a selected target care type being the selected target care type from the terminal device to the central processing unit after selection of the target care type in accordance with the target care type selection screen.

The save-to-device step may save the care related image in the central processing unit as the care record in association with the selected target person and the selected target care type.

In a case where the imaging step and the save selection screen display step are executed from the state where the standby screen is displayed on the display unit, not only the monitored person to be a target for saving in association with the care related image but also the type of care as a target to be saved in association with the care related image is not determined and thus unknown.

In contrast, in the present aspect, the target care type selection screen for selecting the target care type is displayed on the display unit and when the target care type is selected in accordance with the target care type selection screen, the information representing the selected target care type is automatically transmitted from the terminal device to the central processing unit. In addition, the care related image is saved in the central processing unit as a care record in association with the selected target person and the selected target care type. Therefore, according to the present aspect, it is possible to save the care related image as a care record in the central processing unit in association with the selected target person and the selected target care type.

In the first aspect, it is allowable, for example, to further include:
a monitored person selection screen display step of displaying, on the display unit, a monitored person selection screen that prompts selection of the monitored person as a care recipient in order to input the care record; and
a selected monitored person transmission step of transmitting information representing a selected monitored person being the monitored person who has been selected, from the terminal device to the central processing unit after selection of the monitored person being the care recipient in accordance with the monitored person selection screen.

After the information representing the selected monitored person is transmitted from the terminal device to the central processing unit, when the imaging step and the save selection screen display step are executed and saving the care related image in the central processing unit is selected in accordance with the save selection screen, the save-to-device step may save the care related image in the central processing unit as the care record in association with the selected monitored person.

In the present aspect, in order to input a care record, the monitored person selection screen that prompts selection of a monitored person as a care recipient is displayed on the display unit. When the monitored person as a care recipient is selected in accordance with the monitored person selection screen, information representing the selected monitored person who is the monitored person is automatically transmitted from the terminal device to the central processing unit.

In a case where the imaging step and the save selection screen display step are executed after the information representing the selected monitored person is transmitted from the terminal device to the central processing unit, the monitored person as a target to be saved in association with the care related image is highly likely to be a selected monitored person. Therefore, in the present aspect, after the information representing the selected monitored person is transmitted from the terminal device to the central processing unit, when the imaging step and the save selection screen display step are executed and saving the care related image in the central processing unit in accordance with the save selection screen is selected, the care related image is saved in the central processing unit as the care record in association with the selected monitored person. Therefore, according to the present aspect, there is an advantage that it is not necessary to reselect a monitored person which is a target to be saved in association with the care related image after photographing the care related image.

In the first aspect, it is allowable, for example, to further include:
a monitored person selection screen display step of displaying, on the display unit, a monitored person selection screen that prompts selection of the monitored person as a care recipient in order to input the care record;
a selected monitored person transmission step of transmitting information representing a selected monitored person being the monitored person who has been selected, from the terminal device to the central processing unit after selection of the monitored person being the care recipient in accordance with the monitored person selection screen;
a care type selection screen display step of displaying, on the display unit, a care type selection screen that prompts selection of a type of care given to the monitored person, after transmission of information representing the selected monitored person from the terminal device to the central processing unit;
a selected care type transmission step of transmitting information representing a selected care type from the terminal device to the central processing unit, after selection of the type of care given to the monitored person in accordance with the care type selection screen; and
a care type selection screen redisplay step of redisplaying the care type selection screen on the display unit after the imaging step and the save selection screen display step are executed before selection of the type of care from a state where the care type selection screen is displayed on the display unit and then saving the care related image in the central processing unit has been selected in accordance with the save selection screen.

When the type of care given to the monitored person is selected in accordance with the redisplayed care type selection screen, the selected care type transmission step may transmit information representing the selected care type from the terminal device to the central processing unit.

The save-to-device step may save the care related image as the care record in the central processing unit in association with the selected monitored person and the selected care type.

In the present aspect, in order to input a care record, the monitored person selection screen that prompts selection of a monitored person as a care recipient is displayed on the display unit. When the monitored person as a care recipient is selected in accordance with the monitored person selection screen, information representing the selected monitored person is automatically transmitted from the terminal device to the central processing unit. When the information representing the selected monitored person has been transmitted from the terminal device to the central processing unit, a care type selection screen that prompts selection of the type of care given to the monitored person is displayed on the display unit. When the type of care given to the monitored person is selected in accordance with the care type selection screen, information representing the selected care type is automatically transmitted from the terminal device to the central processing unit.

Before the type of care is selected and from the state where the care type selection screen is displayed on the display unit, the imaging step and the save selection screen display step are executed, when saving the care related image in the central processing unit is selected in accordance with the save selection screen, the care type selection screen is redisplayed on the display unit. When the type of care given to the monitored person is selected in accordance with the redisplayed care type selection screen, information representing the selected care type is automatically transmitted from the terminal device to the central processing unit. The care related image is saved in the central processing unit as a care record in association with the selected monitored person and the selected care type. Therefore, according to the present aspect, there is an advantage that it is not necessary to reselect a monitored person which is a target to be saved in association with the care related image after photographing the care related image. Moreover, it is possible to save the care related image in the central processing unit as a care record in association with both the selected monitored person and the selected care type.

In the first aspect, it is allowable, for example, to further include:
a monitored person selection screen display step of displaying, on the display unit, a monitored person selection screen that prompts selection of the monitored person as a care recipient in order to input the care record;
a selected monitored person transmission step of transmitting information representing a selected monitored person being the monitored person who has been selected from the terminal device to the central processing unit after selection of the monitored person being the care recipient in accordance with the monitored person selection screen;
a care type selection screen display step of displaying, on the display unit, a care type selection screen that prompts selection of a care type which is a type of care given to the monitored person, after transmission of information representing the selected monitored person from the terminal device to the central processing unit;
a selected care type transmission step of transmitting information representing a selected care type from the terminal device to the central processing unit, after selection of the type of care given to the monitored person in accordance with the care type selection screen; and
a recording format screen display step of displaying a recording format screen corresponding to the selected care type on the display unit after transmission of information representing the selected care type from the terminal device to the central processing unit.

After the imaging step and the save selection screen display step are executed and saving the care related image in the central processing unit is selected in accordance with the save selection screen from the state where the recording format screen is displayed on the display unit, the save-to-device step may save the care related image in the central processing unit as the care record in association with the selected monitored person and the selected care type.

In the present aspect, in order to input a care record, the monitored person selection screen that prompts selection of a monitored person as a care recipient is displayed on the display unit. When the monitored person as a care recipient is selected in accordance with the monitored person selection screen, information representing the selected monitored person is automatically transmitted from the terminal device to the central processing unit. When the information representing the selected monitored person has been transmitted from the terminal device to the central processing unit, a care type selection screen that prompts selection of the type of care given to the monitored person is displayed on the display unit. When the type of care given to the monitored person is selected in accordance with the care type selection screen, information representing the selected care type is automatically transmitted from the terminal device to the central processing unit. When the information representing the selected care type has been transmitted from the terminal device to the central processing unit, a recording format screen corresponding to the selected care type is displayed on the display unit.

In a case where the imaging step and the save selection screen display step are executed from the state where the recording format screen is displayed on the display unit, the monitored person to be a target for saving in association with the care related image is the selected monitored person, and the type of care to be saved in association with the care related image is highly likely to be the selected care type. Therefore, in the present aspect, when the imaging step and the save selection screen display step are executed and saving the care related image in the central processing unit in accordance with the save selection screen is selected from the state where the recording format screen is displayed on the display unit, the care related image is saved in the central processing unit as the care record in association with the selected monitored person and the selected care type.

Therefore, according to the present aspect, there is an advantage that it is not necessary to reselect a monitored person or the type of care which is targets to be saved in association with the care related image after photographing the care related image. Moreover, it is possible to save the care related image in the central processing unit as a care record in association with both the selected monitored person and the selected care type.

In the first aspect, it is allowable, for example, to further include:
a monitoring information reception step of receiving, on the terminal device, monitoring information related to the monitored person transmitted from the central processing unit;
an action selection screen display step of displaying, on the display unit, an action selection screen that prompts selection of whether to take action for a related monitored person being the monitored person related to the monitoring information, after reception of the monitoring information on the terminal device; and
a time counting step of counting an elapsed time from a point of selection of taking action for the related monitored person in accordance with the action selection screen.

The save selection screen displayed on the display unit in the save selection screen display step in a case where the imaging step has been executed by a point where the elapsed time reaches a predetermined time may prompt selection of whether to save the care related image in the central processing unit in association with the related monitored person.

In the image transmission step, the care related image may be transmitted from the terminal device to the central processing unit when selection is made to save the care related image in the central processing unit in association with the related monitored person in accordance with the save selection screen.

The save-to-device step may save the care related image in association with the related monitored person as the care record in the central processing unit.

In the present aspect, the monitoring information related to the monitored person transmitted from the central processing unit is received on the terminal device. After the monitoring information has been received on the terminal device, an action selection screen that prompts selection of whether to take action for the related monitored person who is the monitored person related to the monitoring information is displayed on the display unit. When it is selected to take action for the related monitored person in accordance with the action selection screen, an elapsed time from the selected point is counted. The save selection screen displayed on the display unit in the save selection screen display step in a case where the imaging step has been executed by a point where the elapsed time reaches a predetermined time prompts selection of whether to save the care related image in the central processing unit in association with the related monitored person.

When saving of the care related image in the central processing unit in association with the related monitored person is selected in accordance with the save selection screen, the care related image is automatically transmitted from the terminal device to the central processing unit. The care related image is saved in the central processing unit as a care record in association with the related monitored person. Therefore, according to the present aspect, it is sufficient to simply select, after a care related image is photographed, operation of saving the care related image in the central processing unit in association with the related monitored person in accordance with the save selection screen. This produces an advantage that it is not necessary to reselect the monitored person who is a target to be saved in association with the care related image.

In the first aspect, it is allowable, for example, to further include:
a temporary saving step of temporarily saving the care related image in an image memory provided in the terminal device after the care related image has been photographed in the imaging step; and
an erasing step of erasing the care related image temporarily saved in the image memory.

The save selection screen display step may display, on the display unit, the care related image saved in the image memory in the temporary saving step.

The erasing step may erase the care related image saved in the image memory from the image memory after the care related image has been transmitted from the terminal device to the central processing unit in the image transmission step.

In the present aspect, when a care related image has been photographed, the care related image is temporarily saved in the image memory provided in the terminal device. The care related image saved in the image memory is displayed on the display unit. After the care related image has been transmitted from the terminal device to the central processing unit, the care related image saved in the image memory is to be erased from the image memory. For this reason, the care related image is saved only in the central processing unit as a care record without being saved in the terminal device. Therefore, according to the present aspect, it is possible to protect the privacy of the monitored person.

While the present invention has been appropriately and fully described in connection with the embodiments thereof with reference to the accompanying drawings, it is to be noted that various changes and/or modifications are apparent to those skilled in the art. Accordingly, such changes and modifications carried out by those skilled in the art are to be understood as included within the scope of the present invention as defined by the appended claims, unless they depart from the scope of the claims described herein.

Although embodiments of the present invention have been illustrated and described in detail, it is understood that such examples are illustrative and exemplary and not restrictive. The scope of the present invention should be construed by the description of the appended claims.

The entire disclosure of Japanese Patent Application No. 2017-007895 filed on Jan. 19, 2017 is incorporated herein by reference in its entirety.

## Claims

1. An assistance method used for an assistance system that assists a caregiver who gives care to a monitored person, the assistance system including:
a central processing unit that saves a care record that records content of care given to the monitored person by the caregiver; and
a terminal device including an imaging unit that photographs an image, a display unit that displays the image photographed by the imaging unit, and an input unit that inputs the care record, and communicably connected to the central processing unit,
the assistance method comprising:
an imaging step of activating the imaging unit and photographing a care related image being an image related to the content of the care given by the caregiver;
a save selection screen display step of displaying, after photographing of the care related image, the photographed care related image and a save selection screen that prompts selection of whether to save the care related image in the central processing unit, on the display unit;
an image transmission step of transmitting the care related image from the terminal device to the central processing unit after selection of saving of the care related image in the central processing unit in accordance with the save selection screen; and
a save-to-device step of saving the care related image as the care record in the central processing unit after transmission of the care related image.

2. The assistance method according to claim 1, further comprising:
a standby screen display step of displaying a standby screen on the display unit;
a target person selection screen display step of displaying, on the display unit, a target person selection screen for selecting a target person being the monitored person as a target to be saved in association with the care related image after the imaging step and the save selection screen display step are executed from a state where the standby screen is displayed on the display unit, and saving the care related image in the central processing unit is selected in accordance with the save selection screen; and
a target person transmission step of transmitting information representing a selected target person being the target person who has been selected, from the terminal device to the central processing unit after the target person is selected in accordance with the target person selection screen,
wherein the save-to-device step saves the care related image in the central processing unit as the care record in association with the selected target person.

3. The assistance method according to claim 2, further comprising:
a target care type selection screen display step of displaying, on the display unit, a target care type selection screen for selecting a target care type which is a type of care as a target to be saved in association with the care related image after transmission of information representing the selected target person from the terminal device to the central processing unit; and
a selected target care type transmission step of transmitting information representing a selected target care type being the selected target care type from the terminal device to the central processing unit after selection of the target care type in accordance with the target care type selection screen,
wherein the save-to-device step saves the care related image in the central processing unit as the care record in association with the selected target person and the selected target care type.

4. The assistance method according to claim 1, further comprising:
a monitored person selection screen display step of displaying, on the display unit, a monitored person selection screen that prompts selection of the monitored person as a care recipient in order to input the care record; and
a selected monitored person transmission step of transmitting information representing a selected monitored person being the monitored person who has been selected, from the terminal device to the central processing unit after selection of the monitored person being the care recipient in accordance with the monitored person selection screen,
wherein, after the information representing the selected monitored person is transmitted from the terminal device to the central processing unit, when the imaging step and the save selection screen display step are executed and saving the care related image in the central processing unit is selected in accordance with the save selection screen, the save-to-device step saves the care related image in the central processing unit as the care record in association with the selected monitored person.

5. The assistance method according to claim 1, further comprising:
a monitored person selection screen display step of displaying, on the display unit, a monitored person selection screen that prompts selection of the monitored person as a care recipient in order to input the care record;
a selected monitored person transmission step of transmitting information representing a selected monitored person being the monitored person who has been selected, from the terminal device to the central processing unit after selection of the monitored person being the care recipient in accordance with the monitored person selection screen;
a care type selection screen display step of displaying, on the display unit, a care type selection screen that prompts selection of a care type which is a type of care given to the monitored person, after transmission of information representing the selected monitored person from the terminal device to the central processing unit;
a selected care type transmission step of transmitting information representing a selected care type from the terminal device to the central processing unit, after selection of the type of care given to the monitored person in accordance with the care type selection screen; and
a care type selection screen redisplay step of redisplaying the care type selection screen on the display unit after the imaging step and the save selection screen display step are executed before selection of the type of care from a state where the care type selection screen is displayed on the display unit and then saving the care related image in the central processing unit has been selected in accordance with the save selection screen,
wherein, when the type of care given to the monitored person is selected in accordance with the redisplayed care type selection screen, the selected care type transmission step transmits information representing the selected care type from the terminal device to the central processing unit, and
the save-to-device step saves the care related image in the central processing unit as the care record in association with the selected monitored person and the selected care type.

6. The assistance method according to claim 1, further comprising:
a monitored person selection screen display step of displaying, on the display unit, a monitored person selection screen that prompts selection of the monitored person as a care recipient in order to input the care record;
a selected monitored person transmission step of transmitting information representing a selected monitored person being the monitored person who has been selected, from the terminal device to the central processing unit after selection of the monitored person being the care recipient in accordance with the monitored person selection screen;
a care type selection screen display step of displaying, on the display unit, a care type selection screen that prompts selection of a care type which is a type of care given to the monitored person, after transmission of information representing the selected monitored person from the terminal device to the central processing unit;
a selected care type transmission step of transmitting information representing a selected care type from the terminal device to the central processing unit, after selection of the type of care given to the monitored person in accordance with the care type selection screen; and
a recording format screen display step of displaying a recording format screen corresponding to the selected care type on the display unit after transmission of information representing the selected care type from the terminal device to the central processing unit,
wherein, after the imaging step and the save selection screen display step are executed and saving the care related image in the central processing unit is selected in accordance with the save selection screen from the state where the recording format screen is displayed on the display unit, the save-to-device step saves the care related image in the central processing unit as the care record in association with the selected monitored person and the selected care type.

7. The assistance method according to any one of claims 1 to 6, further comprising:
a monitoring information reception step of receiving, on the terminal device, monitoring information related to the monitored person transmitted from the central processing unit;
an action selection screen display step of displaying, on the display unit, an action selection screen that prompts selection of whether to take action for a related monitored person being the monitored person related to the monitoring information, after reception of the monitoring information on the terminal device; and
a time counting step of counting an elapsed time from a point of selection of taking action for the related monitored person in accordance with the action selection screen, after the selection of taking the action,
wherein the save selection screen displayed on the display unit in the save selection screen display step in a case where the imaging step has been executed by a point where the elapsed time reaches a predetermined time prompts selection of whether to save the care related image in the central processing unit in association with the related monitored person,
the image transmission step transmits the care related image from the terminal device to the central processing unit after selection is made to save the care related image in the central processing unit in association with the related monitored person in accordance with the save selection screen, and
the save-to-device step saves the care related image as the care record in the central processing unit in association with the related monitored person.

8. The assistance method according to any one of claims 1 to 7, further comprising:
a temporary saving step of temporarily saving the care related image in an image memory provided in the terminal device after the care related image has been photographed in the imaging step; and
an erasing step of erasing the care related image temporarily saved in the image memory,
wherein the save selection screen display step displays, on the display unit, the care related image saved in the image memory in the temporary saving step, and
the erasing step erases the care related image saved in the image memory from the image memory after the care related image has been transmitted from the terminal device to the central processing unit in the image transmission step.

9. An assistance system that assists a caregiver who gives care to a monitored person,
the assistance system comprising:
a central processing unit that saves a care record that records content of care given to the monitored person by the caregiver; and
a terminal device including an imaging unit that photographs an image, a display unit that displays the image photographed by the imaging unit, and an input unit that inputs the care record, and communicably connected to the central processing unit,
the assistance system further comprising:
a photographing processing unit that activates the imaging unit, photographs a care related image being an image related to the content of the care given by the caregiver, and displays the photographed care related image and a save selection screen that prompts selection of whether to save the care related image in the central processing unit, on the display unit;
an image transmission processing unit that transmits the care related image from the terminal device to the central processing unit after selection of saving of the care related image in the central processing unit in accordance with the save selection screen; and
a save-to-device processing unit that saves the care related image as the care record in the central processing unit after transmission of the care related image.
